(19) Europäisches Patentamt
European Patent Office
Office européen des brevets

(11) Veröffentlichungsnummer: **0 521 463 A2**

(12) # EUROPÄISCHE PATENTANMELDUNG

(21) Anmeldenummer: **92111063.1**

(51) Int. Cl.5: **C07D 473/00**, A61K 31/52

(22) Anmeldetag: **30.06.92**

(30) Priorität: **04.07.91 DE 4122068**

(43) Veröffentlichungstag der Anmeldung: **07.01.93 Patentblatt 93/01**

(84) Benannte Vertragsstaaten: **AT BE CH DE DK ES FR GB GR IT LI LU MC NL PT SE**

(71) Anmelder: **HOECHST AKTIENGESELLSCHAFT**
**Postfach 80 03 20**
**W-6230 Frankfurt am Main 80(DE)**

(72) Erfinder: **Jähne, Gerhard, Dr.**
**Johannesallee 14**
**W-6230 Frankfurt am Main 80(DE)**
Erfinder: **Winkler, Irvin, Dr.**
**in den Eichen 40**
**W-6237 Liederbach(DE)**
Erfinder: **Helsberg, Matthias, Dr.**
**Am Rosengarten 3**
**W-6233 Kelkheim (Taunus)(DE)**
Erfinder: **Hänel, Heinz, Dr.**
**Taunusstrasse 148**
**W-6370 Oberursel(DE)**

(54) **Cyclisch substituierte Cycloalkyltriole, Verfahren und Zwischenprodukfe zu ihrer Herstellung sowie ihre Verwendung als antivirale und antiparasitäre Mittel.**

(57) Verbindungen der Formeln I und II,

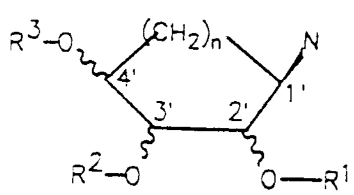

Formel I

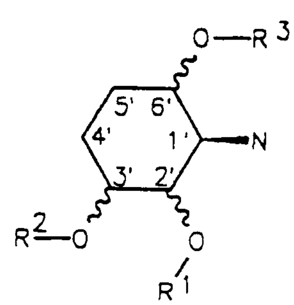

Formel II

in denen die Substituenten N, $R^1$, $R^2$ und $R^3$ sowie n die genannten Bedeutungen haben, besitzen eine antivirale und antiparasitäre Wirkung.

EP 0 521 463 A2

Die vorliegende Erfindung betrifft heterocyclisch substituierte Cycloalkyltriol-Derivate, Verfahren und Zwischenprodukte zur Herstellung dieser Verbindungen sowie ihre Verwendung als antivirale und antiparasitäre Mittel.

Bei den erfindungsgemäßen Verbindungen der Formeln I und II ist ein heterocyclisches System N, das mindestens ein Stickstoffatom enthält, über ein Stickstoffatom mit C-1' des Cycloalkylrestes, im besonderen mit C-1' des Cyclopentyl- oder Cyclohexylrestes, verbunden.

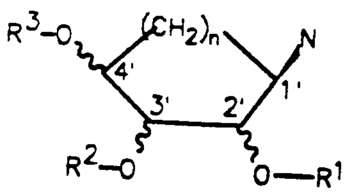

**Formel I**

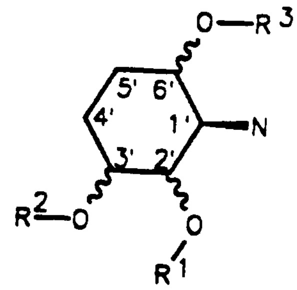

**Formel II**

Im besonderen betrifft die Erfindung Cyclopentan- und Cyclohexantriolderivate von Purinen, Pyrimidinen, Pyridinen, Pyridazinen, Imidazo-[4,5-d]-pyridazinen, Benzimidazolen, Triazolen, Imidazolen und Imiden.

Ferner betrifft die Erfindung die physiologisch verträglichen Salze der genannten Verbindungen mit organischen und anorganischen Säuren, wie z. B. Salze mit Essigsäure, Milchsäure, Apfelsäure, p-Toluolsulfonsäure, Methansulfonsäure, Isäthionsäure, Salzsäure sowie Salze mit organischen oder anorganischen Basen wie z. B. Triethylamin, Pyridin, Ammoniak oder Natronlauge.

Des weiteren betrifft die Erfindung Prodrugs der genannten Verbindungen, wobei Prodrugs in diesem Zusammenhang Derivate meint, die in vivo zu den Muttersubstanzen umgewandelt werden.

Solche Prodrugs können z. B. in vivo hydrolisierbare Ester organischer oder anorganischer Säuren oder Alkyl-, Cycloalkyl- oder Benzylether sein, die in vivo oxidativ zu den entsprechenden Alkoholen rückgespalten werden. Diese Ester oder Ether können sich unabhängig voneinander an jeder der C-2'-, C-3'-, C-4'- oder C-6'-Sauerstoffunktion der Verbindungen der Formeln I und II befinden. Ferner können Amino-, Hydroxy- und Mercaptogruppen des heterocyclischen Systems N in den Formeln I und II in diesem Sinne abgewandelt sein.

Carbocyclische Nukleosidanaloga wie Aristeromycin (Formel A) und Neplanocin A (Formel B) sind natürlich vorkommende Verbindungen, die u. a. antivirale Wirkung aufweisen (siehe z. B. P. Herdewijn et al., J. Med. Chem. 1985, Bd. 28, 1385; E. De Clercq, Antimicrob. Agents Chemother. 1985, Bd. 28, 84).

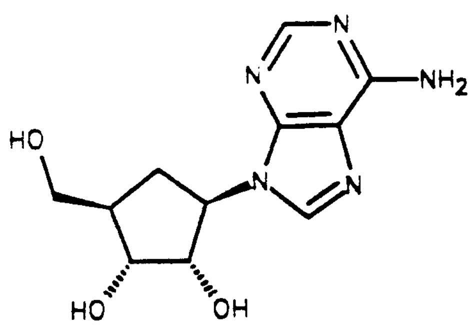

**Formel A**

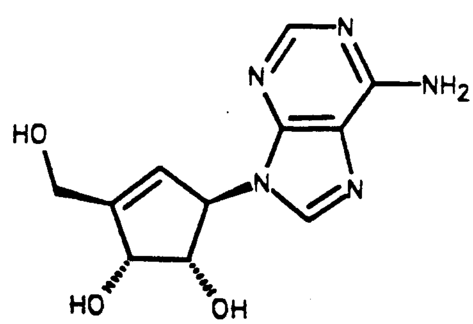

**Formel B**

Derivate dieser Verbindungen, wobei der Hydroxymethylsubstituent in 4'-Position durch Wasserstoff, Alkyl oder Phenyl ersetzt ist, wirken über eine Beeinflussung der Aktivität der S-Adenosyl-L-homocystein Hydrolase auf das Gleichgewicht von S-Adenosyl-L-homocystein/Adenosin/Homocystein ein und erweisen sich als antivirale Wirkstoffe (siehe z. B. M. Hasobe et al., Mol. Pharmacol. 1989, Bd. 36, 490).

Andererseits werden in einer Patentanmeldung (EPA 0368640) Verbindungen der Formel I, wobei N =

Adenin-9-yl oder 8-Aza-Adenin-9-yl, $R^1$ und $R^2$ = Wasserstoff und der Substituent $OR^3$ durch z. B. Wasserstoff oder Alkyl ersetzt ist, als wirksam gegen Infektionen mit Parasiten wie Pneumocystis carinii oder Trypanosoma brucei gambiense beschrieben.

Wir haben nun überraschenderweise gefunden, daß auch solche heterocyclisch substituierten Cyclopentan- und Cyclohexantriolderivate der Formeln I und II, bei denen der 4'-Substituent eine gegebenenfalls substituierte Hydroxyfunktion ist, und deren physiologisch verträgliche Salze eine antivirale und/oder antiparasitäre Wirkung aufweisen.

Erfindungsgegenstand sind demzufolge Verbindungen der Formeln I und II

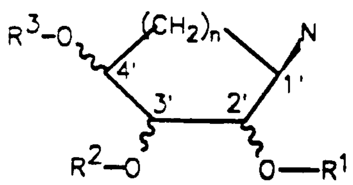

**Formel I**

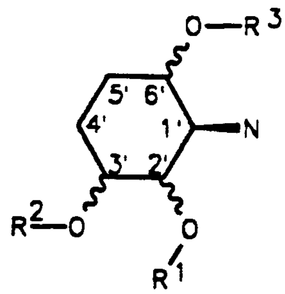

**Formel II**

in denen

A)

N ein über Stickstoff an C-1'gebundener mono- oder bicyclischer Heterocyclus mit mindestens einem Stickstoffatom ist, der unabhängig voneinander mit bis zu drei Amino-, Hydroxy-, Mercapto- und/oder Halogengruppen substituiert sein kann und wobei die Amino-, Hydroxy- und/oder Mercaptogruppen unabhängig voneinander bis zu zweifach mit C1-C6-Alkyl, C1-C6-Acyl-, Benzyl-, Trityl-, Benzyloxymethyl-, Amidingruppen und/oder Phenyl ggfs. seinerseits substituiert mit Halogen, C1-C6-Alkyl, C1-C6-Alkoxy, Amino, C1-C6-Alkylamino oder C1-C12-Dialkylamino substituiert sein können und wobei NH-Funktionen des Heterocyclus mit C1-C6-Acyl-, Benzyl- oder Benzyloxymethylgruppen substituiert sein können, und

n 1 - 4 ist,

und

$R^1$, $R^2$ und $R^3$ unabhängig voneinander Wasserstoff, C1-C8-Alkyl, C3-C8-Alkenyl, C3-C8-Alkinyl, C3-C8-Cycloalkyl, C3-C8-Cycloalkenyl, Benzyl, Trityl, Benzyloxymethyl oder C1-C8-Acyl sind,

oder

$R^1$ und $R^2$ Teil eines 1,3-Dioxa-pentanringes sind, in dessen 2-Position ein oder zwei Wasserstoffatome, unabhängig voneinander mit C1-C8-Alkyl, C2-C16-Dialkyl, C3-C8-Cycloalkyl, C3-C8-Cycloalkenyl, Phenyl, Diphenyl, C1-C8-Alkoxy, C2-C16-Dialkoxy, Dimethylamino, Sauerstoff oder Schwefel substituiert sein können, oder Teil eines cyclischen Sulfits, cyclischen Sulfats, cyclischen Phosphits oder cyclischen Phosphats sind, oder beide Wasserstoffatome ersetzt sind durch die Gruppe $-CH_2-CH_2-CH_2-N(CH_3)-$

und

$R^3$ wie oben angegeben ist, sowie deren physiologisch verträgliche Salze und offensichtliche chemische Äquivalente wobei die Verbindung der Formel C ausgenommen ist.

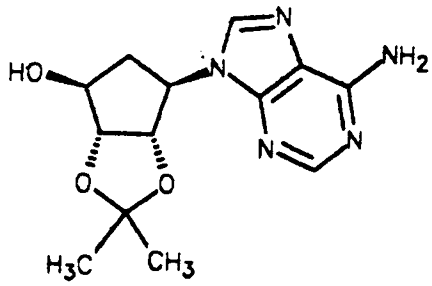

C

Bei Anwendung der in der Nukleosidchemie üblichen Bezeichnungsweise können die Substituenten N, $OR^1$, $OR^2$ und $OR^3$ der Formeln I und II unabhängig voneinander alpha- oder beta-ständig am Carbocyclus sein.

Bevorzugt ist eine Konfiguration 1'$\beta$, 2'$\alpha$, 3'$\alpha$, 4'$\beta$ der Substituenten N, $OR^1$, $OR^2$, $OR^3$ in Verbindungen der Formel I, d. h. die Substituenten an C-1' und C-2' stehen trans, die an C-2' und C-3' stehen cis und die an C-3' und C-4' stehen trans zueinander (Formel Ia), und 1'$\beta$, 2'$\alpha$, 3'$\alpha$, 6'$\beta$ der Substituenten N, $OR^1$, $OR^2$, $OR^3$ in Verbindungen der Formel II, d. h. die Substituenten an C-1' und C-2' stehen trans, die an C-2' und C-3' stehen cis und die an C-6' und C-1' stehen cis zueinander (Formel IIa).

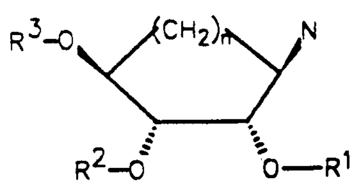

**Formel Ia**

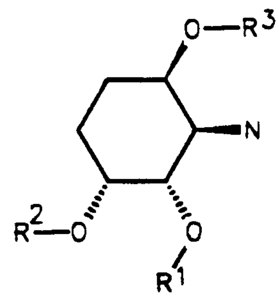

**Formel IIa**

Besonders bevorzugt sind Verbindungen der Formeln Ia und IIa, in denen

B)

N ein über Stickstoff an C-1' gebundenes Purin, Pyrimidin, 8-Azapurin, 1-Deazapurin, 3-Deazapurin, Benzimidazol, Imidazo[4,5-d]pyridazin, Pyridazin, Pyridin, Triazol, Imidazol oder Imid ist, das unabhängig voneinander mit bis zu drei Amino-, Hydroxy-, Mercapto- und/oder Halogengruppen substituiert sein kann und wobei die Amino-, Hydroxy- und/oder Mercaptogruppen unabhängig voneinander bis zu zweifach mit C1-C6-Alkyl-, C1-C6-Acyl-, Benzyl-, Trityl-, Benzyloxymethyl-, Amidingruppen und/oder Phenyl ggfs. seinerseits substituiert mit Halogen, C1-C6-Alkyl, C1-C6-Alkoxy, Amino, C1-C6-Alkylamino oder C1-C12-Dialkylamino substituiert sein können und wobei NH-Funktionen des Heterocyclus mit C1-C3-Acyl-, Benzyl- oder Benzyloxymethylgruppen substituiert sein können,

und

n 1 - 3 ist,

und

$R^1$, $R^2$ und $R^3$ unabhängig voneinander Wasserstoff, C1-C5-Alkyl, C3-C5-Alkenyl, C3-C5-Alkinyl, C3-C5-Cycloalkyl, C3-C5-Cycloalkenyl, Benzyl, Trityl, Benzyloxymethyl oder C1-C7-Acyl sind,

oder

$R^1$ und $R^2$ Teil eines 1,3-Dioxa-pentanringes sind, in dessen 2-Position ein oder zwei Wasserstoffatome, unabhängig voneinander mit C1-C8-Alkyl, C2-C8-Dialkyl, C3-C8-Cycloalkyl, C3-C8-Cycloalkenyl, Phenyl, Diphenyl, C1-C4-Alkoxy, C2-C8-Dialkoxy, Dimethylamino, Sauerstoff

oder Schwefel substituiert sein können, oder Teil eines cyclischen Sulfits, cyclischen Sulfats, cyclischen Phosphits oder cyclischen Phosphats sind, oder beide Wasserstoffatome ersetzt sind durch die Gruppe $-CH_2-CH_2-CH_2-N(CH_3)-$

und

$R^3$ wie oben angegeben ist.

Besonders bevorzugt sind weiterhin Verbindungen der Formeln Ia und IIa, in denen

C)

N ein über Stickstoff an C-1' gebundenes Purin, Pyrimidin, 3-Deazapurin, Imidazo[4,5-d]pyridazin, Pyridazin, Pyridin, Imidazol oder Imid ist, das unabhängig voneinander mit bis zu drei Amino-, Hydroxy-, Mercapto- und/oder Halogengruppen substituiert sein kann und wobei die Amino-, Hydroxy- und/oder Mercaptogruppen unabhängig voneinander bis zu zweifach mit C1-C3-Alkyl-, C1-C3-Acyl-, Benzyl-, Trityl-, Amidingruppen und/oder Phenyl ggfs. seinerseits substituiert mit Halogen, C1-C3-Alkyl, C1-C3-Alkoxy, Amino, C1-C3-Alkylamino oder C1-C6-Dialkylamino substituiert sein können,

und

n 1 oder 2 ist

und

$R^1$, $R^2$ und $R^3$ unabhängig voneinander Wasserstoff, C1-C3-Alkyl, Allyl, Propargyl, Cyclopentyl, Benzyl, Trityl, Benzyloxymethyl oder C1-C4-Acyl sind,

oder

$R^1$ und $R^2$ Teil eines 1,3-Dioxa-pentanringes sind, in dessen 2-Position ein oder zwei Wasserstoffatome, unabhängig voneinander mit C1-C3-Alkyl, C2-C6-Dialkyl, C4-C6-Cycloalkyl, C4-C5-Cycloalkenyl, Phenyl, Diphenyl, C1-C3-Alk oxy, C2-C6-Dialkoxy, Sauerstoff oder Schwefel substituiert sein können, oder Teil eines cyclischen Sulfits, cyclischen Sulfats, cyclischen Phosphits cyclischen Phosphats sind,

und

$R^3$ wie oben angegeben ist.

Ganz besonders bevorzugt sind Verbindungen der Formeln Ia und IIa, in denen

D)

N ein über Stickstoff an C-1' gebundenes Purin, Pyrimidin, 3-Deazapurin, Pyridazin oder Imidazol ist, das unabhängig voneinander mit bis zu drei Amino-, Hydroxy-, Mercapto- und/oder Halogengruppen substituiert ist und wobei die Amino-, Hydroxy- und/oder Mercaptogruppen unabhängig voneinander bis zu zweifach mit C1-C3-Alkyl-, C1-C3-Acyl-, Benzylgruppen, und/oder Phenyl ggfs. seinerseits substituiert mit Chlor, C1-C3-Alkyl, C1-C3-Alkoxy oder Amino substituiert sein können,

und

n 2 ist

und

$R^1$, $R^2$ und $R^3$ unabhängig voneinander Wasserstoff, C1-C3 -Alkyl, Allyl, Propargyl, Cyclopentyl, Benzyl, Trityl, Benzyloxymethyl oder C1-C4-Acyl sind,

oder

$R^1$ und $R^2$ Teil eines 1,3-Dioxa-pentanringes sind, in dessen 2-Position ein oder zwei Wasserstoffatome, unabhängig voneinander mit C1-C3-Alkyl, Cyclopentyl, Phenyl, Diphenyl, C1-C3-Alkoxy, Sauerstoff oder Schwefel substituiert sein können,

und

$R^3$ wie oben angegeben ist.

Eine ganz besondere Bedeutung besitzen die Verbindungen der Formeln Ia und IIa, in denen N die in den Beispielen genannten Bedeutungen hat und $R^1$, $R^2$ und $R^3$ = H sind.

Verfahren zur Herstellung der carbocyclischen Nukleosidanaloga vom Typ der Formeln A und B sind z. B. bei V. E. Marquez, M.-I. Lim, Medicinal Research Reviews Vol. 6, 1 - 40 (1986) beschrieben.

Ein anderer Weg, der sich im Schlüsselschritt einer palladiumkatalysierten Addition von Epoxycyclopenten an Adenin zur Synthese von Aristeromycin (Verbindung der Formel A) bedient, findet sich bei B. M. Trost, G.-H. Kuo und T Benneche, J. Am. Chem. Soc. Vol. 110, 621 (1988) beschrieben. Dieser Weg wird zur Synthese der Adenin-9-yl-, Thymin-1-yl-, Cytosin-1-yl-, und 6-O- Benzylguanin-9-yl-Derivate carbocyclischer Phosphonate in der EPA 0 369 409 ebenfalls beschritten, jedoch ohne daß dabei die erfindungsgemäßen Cyclopentan- und Cyclohexantriolderivate hergestellt oder auf ihre antivirale oder antiparasitäre Wirkung untersucht würden. Lediglich ein Derivat (Verbindung der Formel I a, wobei N = Adenin-9-yl, n = 1 und $R^1$ - $R^3$ = Wasserstoff ist) wird in einer Synthesefolge (B. M. Trost et al., J. Am. Chem. Soc. Vol. 110, 621 (1988)) durchlaufen, ohne daß eine Isolierung oder direkte Charakterisierung erfolgte.

Es wurde nun gefunden, daß

1) eine breite Palette stickstoffhaltiger Heterocyclen mit mindestens einer im Cyclus enthaltenen NH-Funktion diese palladium-katalysierte Reaktion mit Epoxycyclopenten und mit 1,2-Epoxy-cyclohex-3-en unter Bildung des cis-ständigen Adduktes der Formel III, wobei $R^4$ Wasserstoff ist, eingeht,

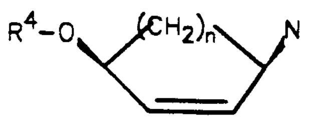

**Formel III**

und daß bei Einsatz von 1,2-Epoxy-cyclohex-3-en das 1,2-cis-ständige Addukt (Verbindungen der Formel IV, wobei $R^4$ Wasserstoff ist) ein weiteres Reaktionsprodukt darstellen kann;

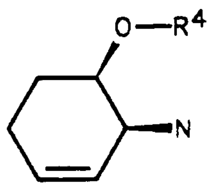

**Formel IV**

2) in diese Reaktion vorteilhaft die silylierten, bevorzugt trimethylsilylierten, oder acylierten, bevorzugt acetylierten, Derivate der stickstoffhaltigen Heterocyclen unter Erhalt von Verbindungen der Formeln III und IV, wobei $R^4$ Trialkylsilyl, bevorzugt Trimethylsilyl, oder Acyl, bevorzugt Acetyl, ist, eingesetzt werden können;

3) aus den Verbindungen der Formeln III und IV durch cis-Hydroxylierung mit z. B. Osmiumtetroxid/N-Methylmorpholin-N-oxid Verbindungen der Formeln I und II, bevorzugt Verbindungen der Formeln Ia und IIa, hergestellt werden können.

Ein weiterer Erfindungsgegenstand ist daher

E)

die Herstellung von Verbindungen der Formeln III und IV, wobei $R^4$ Wasserstoff, Trialkylsilyl, bevorzugt Trimethylsilyl, oder Acyl, bevorzugt Acetyl, und N wie oben definiert ist, dadurch gekennzeichnet, daß ein Äquivalent eines stickstoffhaltigen Heterocyclus, der wie unter A), bevorzugt wie unter B), ganz besonders bevorzugt wie unter C) definiert ist, mit mindestens einer freien NH-Funktion oder mindestens einer silylierten, bevorzugt trimethylsilylierten, oder mindestens einer acylierten, bevorzugt acetylierten, N-Funktion im Heterocyclus, und wobei weitere NH-Funktionen im und Amino-, Hydroxy- und Mercapto-Funktionen am Heterocyclus mit Trimethylsilyl, Acyl, Benzyl, Benzyloxymethyl, Dimethylaminomethyliden oder N-Methyl-2-pyrrolidinyliden substituiert sein können, mit 0.001 - 0.5 Äquivalenten, bevorzugt 0.005 - 0.1 Äquivalenten eines Palladium-(0)-Katalysators wie Tetrakis(triphenylphosphin)palladium(0) oder Tetrakis-(triisopropylphospit)palladium(0), bevorzugt Tetrakis(triisopropylphosphit)palladium(0), welcher in situ aus Palladium-II-acetat, dem jeweiligen Phosphin oder Phosphit und Butyllithium hergestellt werden kann, und mit 1 Äquivalent 1,2-Epoxycycloalk-3-en, bevorzugt Epoxycyclopenten oder 1,2-Epoxy-cyclohex-3-en, in einem Lösungsmittel wie Tetrahydrofuran, Toluol, Dimethylsulfoxid, Dimethylformamid oder N-Methylpyrrolidon-(2) oder Gemischen davon, bevorzugt in Tetrahydrofuran, Dimethylsulfoxid oder Dimethylformamid oder Gemischen davon bei 0°C - 110°C, bevorzugt anfangs bei Raumtemperatur und später bei 80 - 90°C unter einer Schutzgasatmosphäre, beispielsweise Argon, während 2 - 24 Stunden, bevorzugt während 2 - 10 Stunden, zu Verbindungen der obengenannten Formeln III oder/und IV umgesetzt wird,

wobei $R^4$ Wasserstoff ist, wenn in der obigen Reaktion ein stickstoffhaltiger Heterocyclus mit mindestens einer nicht modifizierten NH-Funktion eingesetzt wurde oder $R^4$ Trialkylsilyl, bevorzugt Trimethylsilyl, ist, wenn in der obigen Reaktion ein stickstoffhaltiger Heterocyclus, dessen NH-Funktion trialkylsilyl-, bevorzugt trimethylsilylmodifiziert war eingesetzt wurde oder $R^4$ Acyl, bevorzugt Acetyl, ist, wenn in der obigen Reaktion ein stickstoffhaltiger Heterocyclus, dessen NH-Funktion acyl-, bevorzugt acetyl-modifiziert, war eingesetzt wurde.

Verbindungen der Formeln III und IV, in denen $R^4$ Trialkylsilyl oder Acyl ist, können durch Hydrolyse, Alkoholyse, Ammonolyse oder Aminolyse in Verbindungen der Formeln III und IV, wobei $R^4$ Wasserstoff ist, umgewandelt werden.

Verbindungen der Formeln III und IV, deren eventuell vorhandenen Amino-, Hydroxy- und Mercaptogruppen am Heterocyclus N mit Trimethylsilyl-, Acyl-, Benzyl-, Benzyloxymethyl-, Dimethylaminomethyliden- oder N-Methyl-2-pyrrolidinyliden-Gruppen substituiert sind, können durch Hydrolyse, Alkoholyse, Ammonolyse, Aminolyse oder Hydrogenolyse in Verbindungen der Formeln III und IV, deren Amino-, Hydroxy- und Mercapto-Gruppen frei sind, umgewandelt werden.

Gegenstand der Erfindung ist ferner ein Verfahren

F)

zur Herstellung von Verbindungen der Formeln I und II, bevorzugt von Verbindungen der Formeln Ia und IIa, dadurch gekennzeichnet, daß 1 Äquivalent der Verbindungen der Formeln V oder VI,

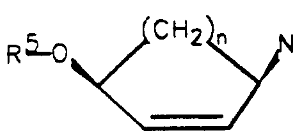

Formel V

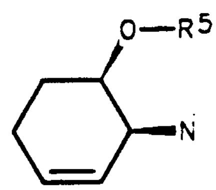

Formel VI

wobei $R^5$ = $R^4$, Alkyl, Benzyl, Trityl oder Benzyloxymethyl ist, und N ein stickstoffhaltiger Heterocyclus wie in A), bevorzugt wie in B), ganz besonders bevorzugt wie in C) definiert, ist, und wobei eventuell vorhandene Amino-, Hydroxy- und Mercapto-Gruppen am Heterocyclus und weitere NH-Gruppen im Heterocyclus mit Trimethylsilyl, C1-C6-Alkyl, C1-C6-Acyl, Benzyl, Trityl, Benzyloxymethyl, Dimethylaminomethyliden oder N-Methyl-2-pyrrolidinyliden substituiert sein können, mit 1 - 5 Äquivalenten, bevorzugt 3 Äquivalenten, N-Methyl-morpholin-N-oxid und einer katalytischen Menge Osmiumtetroxid in Tetrahydrofuran, Aceton oder Wasser oder Gemischen davon bei 0 - 60 ° C, vorzugsweise bei Raumtemperatur, während 2 Stunden bis 4 Tagen, bevorzugt während 1 bis 3 Tagen, umgesetzt wird, wobei Verbindungen der Formeln VII und VIII, bevorzugt Verbindungen der Formeln VIIa und VIIIa, gebildet werden.

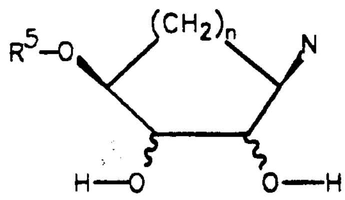

Formel VII

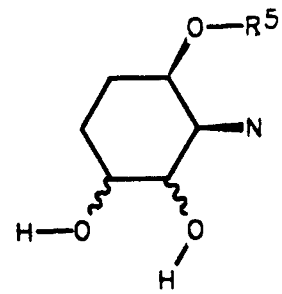

Formel VIII

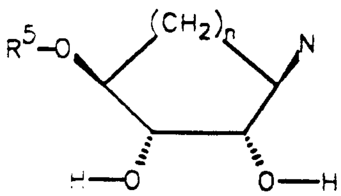

Formel VIIa

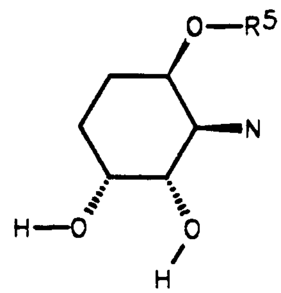

Formel VIIIa

Verbindungen der Formeln VII und VIII, bevorzugt Verbindungen der Formeln VIIa und VIIIa, können ihrerseits durch Standardoperationen in Verbindungen der Formeln I und II, bevorzugt in Verbindungen der Formeln Ia und IIa, wobei die Substituenten N, $R^1$, $R^2$ und $R^3$ die obengenannten Bedeutungen haben, umgewandelt werden.

Weiterhin gehören zum Gegenstand der vorliegenden Erfindung die Verbindungen der obengenannten Formeln III, IV, V, VI, VII, VIIa, VIII und VIIIa, in denen $R^4$ Wasserstoff, C1-C9-Trialkylsilyl, bevorzugt Trimethylsilyl oder C1-C6-Alkyl, C1-C6-Acyl, bevorzugt Acetyl ist und $R^5$ die gleiche Bedeutung wie $R^4$ hat oder C1-C6-, vorzugsweise C1-C3-Alkyl, Benzyl, Trityl oder Benzyloxymethyl ist und N ein stickstoffhaltiger Heterocyclus wie oben unter A), bevorzugt wie unter B), ganz besonders bevorzugt wie unter C) definiert, ist, und wobei eventuell vorhandene Amino-, Hydroxy- und Mercapto-Gruppen am Heterocyclus und weitere NH-Gruppen im Heterocyclus mit Trimethylsilyl, C1-C6-Acyl, Benzyl, Trityl, Benzyloxymethyl, Dimethylaminomethyliden oder N-Methyl-2-pyrrolidinyliden substituiert sein können, mit der Maßgabe, daß in Verbindungen der Formeln III und V $R^4$ und $R^5$ nicht Wasserstoff sind, wenn n = 1 und N = Adenin-9-yl, Thymin-1-yl, Cytosin-1-yl, $N^4$-Dimethylaminomethyliden-cytosin-1-yl, 6-Benzyloxy-2-aminopurin-9-yl, 6-Benzyloxy-2-monomethoxytritylaminopurin-9-yl oder Pyrimidin-2(1H)-on-1-yl ist und daß in Verbindungen der Formel VIIa $R^5$ nicht Wasserstoff ist, wenn n = 1 und N = Adenin-9-yl ist.

Die als Substituenten N an C-1' gebundenen Heterocyclen der obengenannten Formeln I und II können beispielhaft Purin-9-yl, Purin-7-yl, 2-Aminopurin-9-yl, 2-Aminopurin-7-yl, 6-Chlorpurin-9-yl, 6-Chlorpurin-7-yl, 6-Aminopurin-9-yl, 6-Aminopurin-7-yl, 6-Aminopurin-3-yl, Hypoxanthin-9-yl, Hypoxanthin-7-yl, 6-Mercaptopurin-9-yl, 6-Methylthiopurin-9-yl, 6-Dimethylaminopurin-9-yl, 6-Methylaminopurin-9-yl, 6-Isopropoxypurin-9-yl, 2,6-Dichlorpurin-9-yl, 2-Amino-6-chlor-purin-9-yl, 2,6-Diaminopurin-9-yl, Isoguanin-9-yl, Guanin-9-yl, 2-Amino-6-isopropoxypurin-9-yl, 8-Bromguanin-9-yl, 8-Hydroxy-guanin-9-yl, 8-Aminoguanin-9-yl, 6-Amino-1-deaza-purin-9-yl, Uracil-1-yl, Cytosin-1-yl, Thymin-1-yl, 5-Methylcytosin-1-yl, 5-Methyl-4-(1,2,4-triazol-1-yl)-pyrimidin-1H-2-on-1-yl, 5-Ethyluracil-1-yl, 5-Isopropyluracil-1-yl, 5-E-(2-Bromvinyl)-uracil-1-yl, 5-Fluor- oder 5-Chloruracil-1-yl, 6-Phenylthiothymin-1-yl, Pyrimidin-2(1H)-on-1-yl, Benzimidazol-1-yl, 1H-Imidazo[4,5-d]pyridazin-4(5H)-on-1-yl, 5-Methyl-1H-imidazo[4,5-d]pyridazin-4(5H)-on-1-yl, 4-Amino-1H-imidazo[4,5-d]pyridazin-1-yl, 4-Chlor-1H-imidazo[4,5-d]pyridazin-1-yl, Pyridazin-3(2H)-on-2-yl, 4,5-disubstituiertes Pyridazin-3(2H)-on-2-yl, Pyridin-2-on-1-yl, Pyridin-4-on-1-yl, 1,2,4-Triazol-1-yl, 3-Carboxamido-1,2,4-triazol-1-yl, 5-Amino-4-carboxamido-imidazol-1-yl, Phthalimido, Succinimido oder Maleinimido sein, die

unsubstituiert sind oder wobei Amino-, Hydroxy- und Mercaptogruppen am Heterocyclus und NH-Gruppen im Heterocyclus mit Acyl-, Benzyl- oder Amidinfunktionen versehen sein können.

Die als Substituenten genannten Alkylgruppen können verzweigt oder unverzweigt sein.

Beispielhafte Alkylgruppen sind die Methyl-, Ethyl-, Propyl-, Isopropyl-, Butyl-, Isobutyl- oder tert.-Butylgruppe.

Beispielhafte Alkenylgruppen sind die 2-Propen-1-yl-, 3-Buten-1-yl- und die 3-Methyl-2-buten-1-yl-gruppe.

Beispielhafte Alkinylgruppen sind die 2-Propin-1-yl- und die 2-Butin-1-yl-Gruppe.

Beispielhafte Cycloalkylgruppen sind die Cyclobutyl-, Cyclopentyl- und die Cyclohexylgruppe.

Beispielhafte Cycloalkengruppen sind die Cyclopenten-, Cyclohexen- und Cycloheptengruppe.

Die als Substituenten genannten Acylgruppen können aliphatisch, cycloaliphatisch oder aromatisch sein.

Beispielhafte Acylgruppen sind die Formyl-, Acetyl-, Propionyl-, Butyryl-, Isobutyryl-, Pivaloyl-, Valeroyl-, Cyclohexanoyl- oder Benzoylgruppe.

Die als Substituenten genannten Alkoxygruppen können beispielsweise die Methoxy-, Ethoxy-, Propoxy- oder Isopropoxygruppe sein.

Die als Substituenten genannten Amidinfunktionen können acyclisch oder cyclisch sein.

Beispielhafte Amidinfunktionen sind die Dimethylaminomethyliden- oder die N-Methyl-2-pyrrolidinyliden-gruppe.

Die erfindungsgemäßen Verbindungen der Formeln I, Ia, II und IIa können ein oder mehrere chirale Zentren aufweisen. Die Verbindungen liegen in der Regel als Racemate vor; eine Herstellung bzw. Isolierung der reinen Enantiomere ist möglich. Gegenstand der Erfindung sind deshalb sowohl die reinen Enantiomeren als auch Mischungen derselben, wie z. B. das zugehörige Racemat.

Des weiteren betrifft die vorliegende Erfindung Arzneimittel mit einem Gehalt an mindestens einer erfindungsgemäßen Verbindung.

Die erfindungsgemäßen Arzneimittel können enteral (oral), parenteral (intravenös), rektal oder lokal (topisch) angewendet werden. Sie können in Form von Lösungen, Pulvern (Tabletten, Kapseln einschließlich Mikrokapseln), Salben (Cremes oder Gel) oder Suppositorien verabreicht werden. Als Hilfsstoffe für derartige Formulierungen kommen die pharmazeutisch üblichen flüssigen oder festen Füllstoffe und Streckmittel, Lösemittel, Emulgatoren, Gleitstoffe, Geschmackskorrigentien, Farbstoffe und/oder Puffersubstanzen in Frage. Als zweckmäßige Dosierung werden 0.1 - 10, vorzugsweise 0.2 - 8 mg/kg Körpergewicht verabreicht. Sie werden zweckmäßig in Dosierungseinheiten verabreicht, die mindestens die wirksame Tagesmenge der erfindungsgemäßen Verbindungen, z. B. 30 - 300 mg, vorzugsweise 50 - 250 mg enthalten.

Die erfindungsgemäßen Verbindungen können auch in Kombination mit anderen antiviralen oder antiparasitären Mitteln und Immunstimulantien, wie Interferonen, verabreicht werden.

Durch die nachfolgenden Ausführungsbeispiele und durch den Inhalt der Patentansprüche wird die vorliegende Erfindung näher erläutert.

Beispiele:

1.1.1. und 1.1.2.

Verbindung der Formel III, worin N = Adenin-9-yl, n = 1 und $R^4$ = Wasserstoff (Beispiel 1.1.1.) ist und Verbindung der Formel III, worin N = Adenin-3-yl, n = 1 und $R^4$ = Wasserstoff (Beispiel 1.1.2.) ist:

a) Umsatz: von Adenin mit Epoxycyclopenten:

Unter Schutzgas (Argon) werden zu 30 ml wasserfreiem Tetrahydrofuran bei 0°C 89.8 mg (0.4 mMol) Palladium-II-acetat, 1 ml (4.0 mMol) Triisopropylphosphit und 0.58 ml (0.8 mMol) 1.4 M n-BuLi in Hexan gegeben. Die Mischung wird 10 Minuten gerührt, bevor 30 ml wasserfreies Dimethylsulfoxid und 8.66 g (64 mMol) Adenin zugegeben werden. Die Mischung wird weitere 15 Minuten gerührt, bevor über einen Zeitraum von 4 - 5 Stunden eine Lösung von 5.7 g (69.5 mMol) 1,2-Epoxy-cyclopenten in 20 ml wasserfreiem Tetrahydrofuran zugetropft wird. Danach wird die Suspension weitere 3 Stunden bei 0°C gerührt. Nach Entfernung des Eisbades läßt man die Mischung über Nacht Raumtemperatur erreichen. Danach wird die bräunliche Suspension in 300 ml Ethanol gegeben und 30 Minuten bei Raumtemperatur gerührt. Die erhaltene Suspension wird abgesaugt, der Rückstand mit Ethanol gewaschen und getrocknet. Der farblose Rückstand besteht aus 2.75 g Adenin. Das Filtrat wird eingeengt und der Rückstand an neutralem Aluminiumoxid mit Dichlormethan/Methanol 9/1 chromatographiert. Als erste Fraktion werden 2.66 g (28 % d. Th. bezogen auf umgesetztes Adenin) 9-[(1RS, 4SR)-4-Hydroxy-cyclopent-2-en-1-yl]-adenin (Beispiel 1.1.1.) mit dem Schmelzpunkt 176 - 180°C als farblose Kristalle erhalten.

1H NMR (270 MHz, $d_6$-DMSO), δ[ppm]: 8.14 (s, 1H), 8.07 (s, 1H), 7.20 (s, 2H), 6.19 (m, 1H), 5.98 (m, 1H), 5.51 (m, 1H), 5.44 (m, 1H), 4.72 (m, 1H), 2.89 (m, 1H), 1.74 (m, 1H); 13C NMR (67.93 MHz, $d_6$-DMSO), δ [ppm]: 156.01, 152.16, 148.83, 139.28, 139.24, 119.01, 73.74, 57.12, 41.10; UV-VIS [nm, log ⁻]: (0.1 N HCl): 259.5, 4.13; (H2O, pH 6.0): 262.0, 4.18; (Glycin/NaOH-Puffer, pH 11): 261.5, 4.20.

Als zweite Fraktion werden 0.95 g (10 % d. Th. bezogen auf umgesetztes Adenin) 3-[(1RS, 4SR)-4-Hydroxy-cyclopent-2-en-1-yl]adenin (Beispiel 1.1.2.) mit dem Schmelzpunkt 270 - 276° C als farblose Kristalle erhalten. 1H NMR (270 MHz, $d_6$-DMSO), δ[ppm]: 8.31 (s, 1H), 7.93 (s, 2H), 7.74 (s, 1H), 6.63 (d, 1H), 6.25 (m, 1H), 5.97 (m, 1H), 5.61 (d, 1H), 4.70 (t, 1H), 2.92 (m, 1H), 1.92 (m, 1H); 13C NMR (67.93 MHz, $d_6$-DMSO), δ[ppm]: 159.86, 152.10, 151.24, 143.49, 139.94, 130.49, 110.56, 73.56, 60.15, 43.17; UV-VIS [nm, log ⁻]: (0.1 N HCl): 275, 4.25; (H2O, pH 6.0): 274, 4.14; (Glycin/NaOH-Puffer, pH 11): 273, 4.11.

b) Umsatz von persilyliertem Adenin mit Epoxycyclopenten:

Der Umsatz (3 Stunden bei 0° C, dann über Nacht bei Raumtemperatur) von 94.5 g (0.7 Mol) Adenin, das durch Reaktion mit Hexamethyldisilazan und 4 g Ammoniumsulfat in Xylol bei Rückflußtemperatur in das Persilylderivat überführt und in 600 ml trockenem Tetrahydrofuran gelöst wurde, mit dem Palladium-O-katalysator (3.15 g (0.014 Mol) Palladium-II-acetat, 38.1 ml (0.14 Mol) Triisopropylphosphit und 17.5 ml (0.028 Mol) 1.6 molare Lösung von Butyllithium in n-Hexan) und einer Lösung von 68 g rohem Epoxycyclopenten in 350 ml tockenem Tetrahydrofuran unter Argon-Schutzgas in 500 ml trockenem Tetrahydrofuran lieferte nach der Hydrolyse mit Methanol neben 19.1 g nicht-umgesetztem Adenin nach der Chromatographie (Kieselgel, Dichlormethan/Methanol 9/1) 76.7 g (63.3 % d. Th. bezogen auf umgesetztes Adenin) 9-[(1RS, 4SR)-4-Hydroxy-cyclopent-2-en-1-yl]adenin (Beispiel 1.1.1.) und 30.9 g (25.5 % d. Th. bezogen auf umgesetztes Adenin) 3-[(1RS, 4SR)-4-Hydroxy-cyclopent-2-en-1-yl]adenin (Beispiel 1.1.2.).

c) Umsetzung der Verbindung aus Beispiel 1.5.1. mit $NH_3$:

0.5 g (2.1 mMol) der Verbindung aus Beispiel 1.5.1. werden in 25 ml n-Propanol gelöst. Es werden 50 ml flüssiges $NH_3$ zugegeben und die Mischung wird im Autoklaven bei einem Druck von 50 bar Stickstoff 10 Stunden bei 100° C behandelt. Das Reaktionsprodukt wird eingeengt und der Rückstand über Kieselgel mit Dichlormethan/Methanol 9/1 chromatographiert. Man erhält 0.38 g (83.4 % d. Th.) 9-[(1RS, 4SR)-4-Hydroxy-cyclopent-2-en-1-yl]adenin mit einem Schmelzpunkt von 180° C.

1.2.

Verbindung der Formel III, worin N = [$N^6$-(N-Methyl-2-pyrrolidin-yliden)]adenin-9-yl, n = 1 und $R^4$ = Wasserstoff ist:

a) Umsatz von $N^6$-(N-Methyl-2-pyrrolidin-yliden)adenin mit Epoxycylopenten:

Unter Stickstoff-Schutzgas werden in 40 ml trockenem Tetrahydrofuran bei 0° C 224 mg (1 mMol) Palladium-II-acetat, 2.5 ml (10 mMol) Triisopropylphosphit und 1.5 ml 1.6 molare n-Butyllithium-Lösung in n-Hexan (2 mMol) zusammengegeben und gerührt. Nach der Zugabe von 30 ml trockenem Dimethylsulfoxid werden 4.32 g (20 mMol) $N^6$-(N-Methyl-pyrrolidin-yliden)adenin (hergestellt durch Reaktion von Adenin mit dem Diethylacetal von N-Methyl-pyrrolidon-(2) in 2-Propanol; beiges Pulver, Fp.: 248 - 251° C) zugegeben; sodann wird über eine Stunde eine Lösung von 1.64 g (20 mMol) Epoxycyclopenten in 20 ml trockenem Tetrahydrofuran zugetropft. Die Lösung wird 12 Stunden bei Raumtemperatur gerührt, danach eingeengt und über Kieselgel (Dichlormethan/Methanol 9/1) chromatographiert. In regiospezifischer Weise werden 1.62 g (27 % d. Th.) $N^6$-(N-Methyl-2-pyrrolidin-yliden)-9-[(1RS,4SR)-4-hydroxy-cyclopent-2-en-1-yl]adenin vom Schmelzpunkt 135 - 138° C erhalten. 1H NMR (270 MHz, $d_6$-DMSO), δ[ppm]: 8.42 (s, 1H), 8.16 (s, 1H), 6.20 (m, 1H), 6.00 (m, 1H), 5.50 (m, 1H), 5.46 (d, 1H), 4.73 (m, 1H), 3.50 (t, 2H), 3.04 (s, 3H), 2.90 (m, 1H), 2.85 (t, 2H), 1.97 (p, 2H), 1.74 (m, 1H). Als Nebenprodukt (20 mg, 0.3 % d. Th.) wird $N^6$-(N-Methyl-2-pyrrolidin-yliden)-9-(cyclopent-1-en-3-on-1-yl)adenin mit dem Schmelzpunkt 200 - 205° C isoliert. 1H NMR (270 MHz, $CDCl_3$), δ[ppm]: 8.64 (s, 1H), 8.13 (s, 1H), 7.19 (m, 1H), 3.53 (t,2H), 3.34 (m, 2H), 3.20 (s, 3H), 3.07 (t, 2H), 2.65 (m, 2H), 2.10 (p, 2H).

b) Umsatz von silyliertem $N^6$-(N-Methyl-pyrrolidin-yliden)-adenin mit Epoxycyclopenten:

Setzt man in obige Reaktion trimethylsilyliertes $N^6$-(N-Methyl-pyrrolidin-yliden)adenin (hergestellt durch Reaktion mit N,O-Bis-(trimethylsilyl)acetamid) ein, so werden nach Reaktion mit Tetrabutylammoniumfluorid und nach Chromatographie 29 % d. Th. $N^6$-(N-Methyl-pyrrolidin-yliden)-9-[(1RS, 4SR)-4-hydroxy-cyclopent-2-en-1-yl]adenin in regiospezifischer Weise erhalten.

c) Umsatz von 9-[(1RS, 4SR)-4-Hydroxy-cyclopent-2-en-1-yl]adenin mit dem Diethylacetal von N-Methyl-pyrrolidon-(2):

Die Verbindung des Beispiels 1.2. kann auch gewonnen werden, wenn man 9-[(1RS, 4SR)-4-Hydroxy-

cyclopent-2-en-1-yl]adenin mit dem Diethylacetal des N-Methyl-pyrrolidon-(2) in Dichlormethan umsetzt. Ausbeute nach Chromatographie an Kieselgel mit Dichlormethan/Methanol 9/1 : 98 % d. Th.

1.3.

Verbindung der Formel III, worin N = Adenin-$N^1$-Oxid-9-yl, n = 1 und $R^4$ = Wasserstoff ist (als m-Chlorbenzoesäuresalz):
0.9 g (4.15 mMol) der Verbindung aus Beispiel 1.1.1. werden mit 0.84 g (4.15 mMol) 85%iger m-Chlorperbenzoesäure bei Raumtemperatur 2 Tage in 50 ml Dichlormethan gerührt. Der Niederschlag wird abgesaugt, mit 50 ml Diethylether verrührt und erneut abgesaugt. Der feste Rückstand wird in 2N HCl gelöst, die saure wäßrige Lösung wird mehrmals mit Diethylether ausgeschüttelt und schließlich vollständig eingeengt. Der Rückstand wird in mehreren Portionen mit Ethanol heiß extrahiert. Die Ethanolextrakte werden eingeengt und getrocknet. Man erhält 0.44 g (45.5 % d. Th.) des m-Chlorbenzoesäuresalzes des $N^1$-Oxids von 9-[(1RS,4SR)-4-Hydroxy-cyclo-pent-2-en-1-yl]adenin mit dem Schmelzpunkt 198 - 200 ° C.
1H NMR (270 MHz, $d_6$-DMSO), δ[ppm]: 8.66 (s, 1H), 8.27 (s, breit, 1H), 8.22 (s, 1H), 7.89 (m, 2H), 7.70 (m, 1H), 7.53 (m, 1H), 6.20 (m, 1H), 6.04 (m, 1H), 5.45 (m, 1H), 5.28 (s, 1H), 4.72 (m, 1H), 2.91 (m, 1H), 1.73 (m, 1H).

1.4.

Verbindung der Formel III, wobei N = Hypoxanthin-9-yl, n = 1 und $R^4$ = Wasserstoff ist:
6.51 g (30 mMol) des Adeninderivates aus Beispiel 1.1.1. werden mit 15.3 g (180 mMol) Natriumnitrit in einer Mischung aus 150 ml Wasser, 15 ml Eisessig und 30 ml 1N Salzsäure gelöst und 2 Tage bei Raumtemperatur gerührt. Die resultierende Suspension wird auf ca. 1/3 des ursprünglichen Volumens eingeengt, der Niederschlag wird abgesaugt und aus Ethanol/Wasser 2/1 umkristallisiert. Man erhält 5.02 g (76.8 % d. Th.) 9-[(1RS, 4SR)-4-Hydroxy-cyclopent-2-en-1-yl]hypoxanthin mit dem Schmelzpunkt 274 - 276 ° C (Zers.). 1H NMR (270 MHz, $d_6$-DMSO), δ[ppm]: 12.29 (s, 1H), 8.06 (s, 1H), 8.00 (s, 1H), 6.18 (m, 1H), 6.00 (m, 1H), 5.43 (m, 1H), 5.28 (d, 1H), 4.72 (m, 1H), 2.70 (m, 1H), 1.70 (m, 1H).

1.5.1. und 1.5.2.

Verbindungen der Formel III, wobei N = 6-Chlorpurin-9-yl (Beispiel 1.5.1.) oder 6-Chlorpurin-7-yl (Beispiel 1.5.2.) und n = 1 und $R^4$ = Wasserstoff ist:
Trimethylsilyliertes 6-Chlorpurin (hergestellt aus 50 g (0.32 Mol) 6-Chlorpurin) werden mit 5 Mol-% des oben beschriebenen Palladium-0-katalysators und einer äquivalenten Menge Epoxycyclopenten 24 Stunden bei Raumtemperatur in Tetrahydrofuran gerührt. Die Reaktionslösung wird 1.5 Stunden mit Methanol erhitzt, der gebildete Niederschlag abgesaugt, die resultierende Lösung eingeengt und über Kieselgel mit Dichlormethan/Methanol 20/1 chromatographiert. Man erhält 18.9 g (25 % d. Th.) 6-Chlor-9-[(1RS,4SR)-4-hydroxy-cyclopent-2-en-1-yl]purin (Beispiel 1.5.1.) als gelbliche Kristalle mit dem Schmelzpunkt 118 ° C. 1H-NMR (270 MHz, $d_6$-DMSO), δ[ppm]: 8.80 (s, 1H), 8.62 (s, 1H), 6.25 (m, 1H), 6.08 (m, 1H), 5.60 (m, 1H), 5.31 (d, 1H), 4.76 (m, 1H), 2.95 (m, 1H), 1.81 (m, 1H).

In einer zweiten Fraktion werden 34.2 g (45.2 % d. Th.) 6-Chlor-7-[(1RS, 4SR)-4-hydroxy-cyclopent-2-en-1-yl]-purin (Beispiel 1.5.2.) als gelbe Kristalle mit dem Schmelzpunkt 177 ° C erhalten. 1H NMR (270 MHz, $d_6$-DMSO), δ[ppm]: 8.81 (s, 1H), 8.68 (s, 1H), 6.30 (m, 1H), 6.18 (m, 1H), 5.84 (m, 1H), 5.27 (d, 1H), 4.75 (m, 1H), 2.99 (m, 1H), 1.73 (m, 1H).

Die Verbindung des Beispiels 1.5.1. kann regioisomerenrein (d. h. ohne Verunreinigung durch das $N^7$-Isomer) erhalten werden, wenn 6-Chlorpurin mit 5 Mol-% des oben beschriebenen Palladium-0-katalysators und mit einer äquivalenten Menge Epoxycyclopenten in Tetrahydrofuran bei Raumtemperatur 2 Tage gerührt wird. Das so erhaltene Rohprodukt wird in 2N Natronlauge gelöst, mehrmals mit Essigester ausgeschüttelt, die organische Phase über Natriumsulfat getrocknet, eingeengt und über Kieselgel mit Dichlormethan/Methanol 20/1 chromatographiert. Auf diese Art wird 6-Chlor-9-[(1RS, 4SR)-4-hydroxy-cyclopent-2-en-1-yl]purin in einer Ausbeute von 74 % erhalten.

1.6.

Verbindung der Formel III, wobei N = Adenin-7-yl, n = 1 und $R^4$ = Wasserstoff ist:
0.5 g (2.1 mMol) der Verbindung der Beispiels 1.5.2. werden in 30 ml mit Ammoniakgas gesättigtem n-Pentanol gelöst und 10 Stunden im Autoklaven unter einem Druck von 50 bar bei 100 ° C gerührt. Das zur

Trockne eingeengte Rohprodukt wird an Kieselgel mit Dichlormethan/Methanol 7/3 chromatographiert und liefert 0.32 g (70.2 % d. Th.) 7-[(1RS, 4SR)-4-Hydroxy-cyclopent-2-en-1-yl]adenin als farblose Kristalle mit dem Schmelzpunkt 195 - 197 ° C.
1H-NMR (270 MHz, $d_6$-DMSO), δ[ppm]: 8.19 (Zentrum zweier nicht vollständig aufgelöster Singulette, 2H), 6.93 (s, 2H), 6.22 (m, 1H), 6.13 (m, 1H), 5.68 (m, 1H), 5.38 (d, 1H), 4.72 (m, 1H), 2.98 (m, 1H), 1.52 (m, 1H). 13C NMR (67.93 MHz, $d_6$-DMSO), δ[ppm]: 159.86, 152.10, 151.24, 143.49, 139.94, 130.49, 110.56, 73.56, 60.15, 43.17.

1.7.

Verbindung der Formel III, wobei N = 6-(2,2-Diphenyl-ethylamino)-purin-9-yl, n = 1 und $R^4$ = Wasserstoff ist:
4.52 g (0.02 Mol) der Verbindung aus Beispiel 1.5.1. werden mit 5.91 g (0.03 Mol) 2,2-Diphenylethylamin und 3.03 g (0.03 Mol) Triethylamin in 150 ml Ethanol 6 Stunden zum Rückfluß erhitzt. Die Reaktionslösung wird eingeengt, in 200 ml Dichlorethan gelöst, mehrmals mit Wasser ausgeschüttet, die organische Phase über Natriumsulfat getrocknet, eingeengt und der Rückstand über Kieselgel mit Dichlormethan/Methanol 9/1 chromatographiert. Man erhält 6 g (75.7 % d. Th.) 6-(2,2-Diphenylethylamino)-9-[(1RS,4SR)-4-hydroxy-cyclopent-2-en-1-yl]purin als hellbraune Kristalle mit einem Schmelzpunkt von 165 - 169 ° C (nach Kristallisation aus 2-Propanol/n-Hexan 1/1). 1H NMR (270 MHz, $d_6$-DMSO), δ[ppm]: 8.28 (s, 1H), 8.03 (s, 1H), 7.72 (s, 1H), 7.38 - 7.14 (m, 10H), 6.18 (m, 1H), 5.96 (m, 1H), 5.51 (d, 1H), 5.44 (m, 1H), 4.71 (m, 1H), 4.60 (t, 1H), 4.13 (m, 2H), 2.89 (m, 1H), 1.74 (m, 1H).

1.8.

Verbindung der Formel III, wobei N = 2-Amino-6-chlor-purin-9-yl, n = 1 und $R^4$ = Wasserstoff ist:
39.9 g (0.235 Mol) 2-Amino-6-chlorpurin werden in einer Mischung aus 100 ml Tetrahydrofuran und 100 ml Dimethylsulfoxid mit 5 Mol-% des oben beschriebenen Katalysators und mit einer äquivalenten Menge Epoxycyclopenten 90 Stunden bei Raumtemperatur gerührt. Die erhaltene Suspension wird filtriert (1.7 g 2-Amino-6-chlorpurin); das Filtrat wird eingeengt und über Kieselgel mit Dichlorethan/Methanol 9/1 chromatographiert. Neben weiteren 3.2 g 2-Amino-6-chlorpurin werden 48.5 g (93.4 % d. Th. bez. auf umgesetztes 2-Amino-6-chlorpurin) 2-Amino-6-chlor-9-[(1RS, 4SR)-4-hydroxy-cyclopent-2-en-1-yl)purin als gelbes Öl erhalten. Nach einiger Zeit kristallisiert das Öl und die Substanz weist dann einen Schmelzpunkt von 148 bis 151 ° C auf. 1H NMR (270 MHz, $d_6$-DMSO), δ[ppm]: 8.04 (s, 1H), 6.91 (s, 2H), 6.20 (m, 1H), 6.00 (m, 1H), 5.32 (m, 1H), 5.25 (d, 1H), 2.86 (m, 1H), 1.68 (m, 1H).

1.9.

Verbindung der Formel III, wobei N = 2,6-Diaminopurin-9-yl, n = 1 und $R^4$ = Wasserstoff ist:
6.0 g (23.8 mMol) der Verbindung des Beispiels 1.8. werden in 80 ml Formamid suspendiert, und die Mischung wird auf 100 ° C erwärmt. Durch die resultierende Lösung wird 7 Stunden gasförmiges Ammoniak durchgeleitet. Die Reaktionslösung wird abgekühlt, mit 60 ml einer gesättigten Lösung von Salzsäuregas in Methanol angesäuert und mit 800 ml Aceton versetzt. Der Niederschlag wird abgesaugt, in 200 ml Wasser gelöst mit 50 ml 2N Ammoniaklösung versetzt und vollständig eingeengt. Der Rückstand wird mehrmals mit heißem Aceton extrahiert; die Acetonextrakte werden filtriert, und das Filtrat wird vollständig eingeengt. Der Rückstand wird mit Diethylether verrührt, abgesaugt und getrocknet. Man erhält 3.91 g (70.8 % d. Th.) 2,6-Diamino-9-[(1RS, 4SR)-4-hydroxy-cyclopent-2-en-1-yl]purin mit dem Schmelzpunkt 167 - 170 ° C. 1H NMR (60 MHz, $d_6$-DMSO), δ[ppm]: 7.70 (s, 1H), 7.03 (s, 2H), 6.12 (m, 5H), 5.30 (m, 1H), 4.73 (m, 1H), 2.88 (m, 1H), 1.68 (m, 1H).

1.10.

Verbindung der Formel III, wobei N = 2-Amino-6-mercapto-purin-9-yl, n = 1 und $R^4$ = Wasserstoff ist:
6.0 g (23.8 mMol) der Verbindung aus Beispiel 1.8. werden mit 2.0 g (26.2 mMol) Thioharnstoff in 80 ml 2-Propanol suspendiert, gerührt und 45 Minuten zum Rückfluß erhitzt. Die erhaltene Suspension wird filtriert; der Rückstand wird in 30 ml heißem Wasser mit Aktivkohle versetzt, filtriert und abgekühlt. Es kristallisieren 2.76 g 2-Amino-6-mercapto-9-[(1RS, 4SR)-4-hydroxy-cyclo-pent-2-en-1-yl]purin mit einem Schmelzpunkt >250 ° C. Chromatographie der eingeengten Mutterlaugen an Kieselgel mit Dichlormethan/Methanol 9/1 liefern weitere 1.03 g Produkt, so daß die Gesamtausbeute 63.9 % d. Th. beträgt. 1H NMR (60 MHz, $d_6$-

DMSO), δ[ppm]: 12.5 (s, 1H), 8.45 (s, 1H), 7.20 (s, 2H), 6.15 (m, 2H), 5.83 - 5.12 (m, 2H), 4.75 (m, 1H), 2.85 (m, 1H), 1.73 (m, 1H).

1.11.

Verbindung der Formel III, wobei N = 2-Aminopurin-9-yl, n = 1 und $R^4$ = Wasserstoff ist:

a) 3.74 g (15 mMol) der Verbindung des Beispiels 1.10. werden in 600 ml trockenem Ethanol suspendiert, mit 15 g Raney-Nickel versetzt und 2 Stunden bei Rückflußtemperatur gerührt. Die abgekühlte Suspension wird filtriert, der Rückstand mit heißem Ethanol gewaschen, die vereinigten Ethanollösungen eingeengt, der Rückstand mit Diethylether/Aceton 10/1 verrührt und filtriert. Der Rückstand wird mit Diethylether gewaschen und getrocknet. Man erhält 1.34 g (41.2 % d. Th.) 2-Amino-9-[(1RS, 4SR)-4-hydroxy-cyclopent-2-en-1-yl]purin vom Schmelzpunkt 144 - 147 ° C. 1H NMR (60 MHz, $d_6$-DMSO), δ[ppm]: 8.67 (s, 1H), 8.05 (s, 1H), 6.53 (s, 2H), 6.13 (m, 2H), 5.58 - 5.20 (m, 2H), 4.77 (m, 1H), 2.92 (m, 1H), 1.70 (m, 1H).

b) 3.74 g (15 mMol) der Verbindung des Beispiels 1.8. und 7 g Zinkpulver werden in 150 ml Wasser suspendiert. Die Suspension wird auf Rückflußtemperatur erhitzt, und unter Rühren werden über einen Zeitraum von 2 Stunden 2 ml konzentrierte wäßrige Ammoniaklösung zugetropft. Die abgekühlte Suspension wird filtriert, der Filterrückstand wird mit Methanol gewaschen, das Filtrat wird eingeengt und über Kieselgel mit Dichlormethan/Methanol 5/1 chromatographiert. Man erhält 2.66 g (81.7 % d. Th.) 2-Amino-9-[(1RS,4SR)-4-hydroxy-cyclopent-2-en-1-yl]purin vom Schmelzpunkt 147 - 149 ° C. Als Nebenprodukt werden 0.22 g (6.3 % d. Th.) der Diaminopurinverbindung des Beispiels 1.9. isoliert.

1.12.1. und 1.12.2.

Verbindungen der Formel III, wobei N = Guanin-9-yl, n = 1 und $R^4$ = Wasserstoff ist (Beispiel 1.12.1.) und wobei N = Guanin-7-yl, n = 1 und $R^4$ = Wasserstoff ist (Beispiel 1.12.2.):

a) 105.8 g (0.7 Mol) Guanin werden in 600 ml trockenem Xylol 2 Tage mit 500 ml Hexamethyldisilazan und 4 g Ammoniumsulfat zur Pertrimethylsilylverbindung umgesetzt. Die ölige Silylguaninverbindung wird in 400 ml trockenem Tetrahydrofuran gelöst und zu einer Lösung des in situ wie oben beschrieben hergestellten Palladium-0-katalysators (5 Mol-%) in 450 ml Tetrahydrofuran bei 0 ° C zugetropft. Danach wird über einen Zeitraum von 2 - 3 Stunden eine Lösung der äquivalenten Menge Epoxycyclopenten in 200 ml Tetrahydrofuran zugetropft. Die resultierende Lösung wird 3 Tage bei Raumtemperatur gerührt. Man fügt 400 ml Methanol zu und erhitzt 1 Stunde lang zum Rückfluß. Dabei entsteht eine sehr voluminöse Fällung, die nach weiterem Rühren in eine homogene Suspension überführt werden kann. Die so erhaltene Suspension wird filtriert. Aus dem Filtrat kristallisiert 4.7 g eines Gemisches der Verbindungen 1.12.1. und 1.12.2. Der Filterrückstand wird mit 1.2 l Wasser verrührt und erneut abgesaugt. Dieser Filterrückstand wird mit Ethanol gewaschen und dann in mehreren Portionen mit einem l Ethanol ausgekocht. Beim Abkühlen liefert diese ethanolische Lösung 4.75 g 9-[(1RS, 4SR)-4-Hydroxy-cyclopent-2-en-1-yl]guanin als beige Kristalle mit einem Schmelzpunkt von 265 - 273 ° C (Zers.) (Verbindung des Beispiels 1.12.1.). 1H NMR (270 MHz, $d_6$-DMSO), δ [ppm]: 10.56 (s, 1H), 7.60 (s, 1H), 6.42 (s, 2H), 6.15 (m, 1H), 5.95 (m, 1H), 5.24 (d, 1H), 5.20 (m, 1H), 4.70 (m, 1H), 2.82 (m, 1H), 1.60 (m, 1H). Wird der Filterrückstand erneut mit 1.5 l Wasser ausgekocht, so kristallisieren aus dem wäßrigen Filtrat 5.0 g 7-[(1RS, 4SR)-4-Hydroxy-cyclopent-2-en-1-yl]guanin mit dem Schmelzpunkt >310 ° C (Verbindung des Beispiels 1.12.2.). 1H NMR (270 MHz $d_6$-DMSO), δ[ppm]: 10.80 (s, 1H), 7.84 (s, 1H), 6.15 (m, 1H), 6.13 (s, 2H), 6.01 (m, 1H), 5.61 (m, 1H), 5.18 (d, 1H), 4.68 (m, 1H), 2.88 (m, 1H), 1.60 (m, 1H). Der verbleibende Filterrückstand von 82.7 g besteht aus einem Gemisch der Verbindungen des Beispiels 1.12.1. und 1.12.2.

b) 2 g (7.9 mMol) der Verbindung des Beispiels 1.8. werden in 50 ml 2N wäßriger Salzsäure suspendiert und unter Rühren 6 Stunden auf ca. 70 ° C erwärmt. Die erhaltene gelbe Suspension wird mit Eis versetzt und vorsichtig durch portionsweise Zugabe von festem Natriumcarbonat auf pH 7 - 8 eingestellt. Der Niederschlag wird abgesaugt, mit wenig kaltem Wasser gewaschen und getrocknet. Man erhält 1.3 g (70.2 % d. Th.) 9-[(1RS, 4SR)-4-Hydroxy-cyclopent-2-en-1-yl]-guanin (Verbindung des Beispiels 1.12.1.) vom Schmelzpunkt 270 - 273 ° C.

c) 19.3 g (0.1 Mol) $N^2$-Acetylguanin werden mit Hexamethyldisilazan und einer katalytischen Menge Ammoniumsuffat in Xylol zur entsprechenden pertrimethylsilylierten Verbindung umgesetzt. Diese wird in Tetrahydrofuran mit 5 Mol-% des wie oben beschriebenen in situ hergestellten Palladium-0-katalysators und einer äquivalenten Menge Epoxycyclopenten bei Raumtemperatur während 16 Stunden umgesetzt. Man fügt Methanol und Wasser zu und erhitzt die Mischung 3 Stunden unter Rückfluß. Die Mischung

wird vollständig eingeengt, in 2H Kaliumcarbonat gelöst und mit Essigester ausgeschüttelt. Die wäßrige Phase wird mit Essigsäure neutralisiert, vollständig eingeengt und mit Essigsäureanhydrid und katalytischen Mengen N,N-Dimethylaminopyridin in Dichlormethan acetyliert. Das Reaktionsprodukt wird über Kieselgel mit Dichlormethan/Methanol 20/1 chromatographiert. Man erhält 7.42 g (27 % d. Th.) $N^2$-Acetyl-7-[(1RS, 4SR)-4-acetoxy-cyclopent-2-en-1-yl]guanin (N, O-Diacetylverbindung des Beispiels 1.12.2.) mit dem Schmelzpunkt 219 - 220 °C (Zers.).
1H NMR (270 MHz, $d_6$-DMSO) δ[ppm]: 12.12 (s, 1H), 11.59 (s, 1H), 8.03(s, 1H), 6.34 (m, 1H), 6.27 (m, 1H), 5.78 (m, 1H), 5.60 (m, 1H), 3.04 (m, 1H), 2.18 (s, 3H), 2.01 (s, 3H), 1.87 (m, 1H). Diese Verbindung wird in Methanol mit 40 %iger wäßriger Methylaminlösung bei Rückflußtemperatur behandelt und liefert 5.3 g (97.2% d. Th.) 7-[(1RS, 4SR)-4-Hydroxy-cyclopent-2-en-1-yl]guanin (Verbindung des Beispiels 1.12.2.) mit einem Schmelzpunkt >310 °C.

1.13.

Verbindung der Formel III, wobei N = $N^2$-Acetyl-6-O-diphenylcarbamoyl-guanin-7-yl, n = 1 und $R^4$ = Wasserstoff ist:
38.8 g (0.1 Mol) $N^2$-Acetyl-6-O-diphenylcarbamoyl-guanin (hergestellt nach R. Zou, M. J. Robins: Can. J. Chem. Vol. 65, 1436 (1987)) werden mit 5 Mol-% des wie oben bebeschriebenen in situ hergestellten Palladium-0-katalysators in einer Mischung aus 200 ml trockenem Tetrahydrofuran und 200 ml trockenem Dimethylsulfoxid bei 0 °C mit 0.15 Mol Epoxycyclopenten versetzt und 15 Stunden bei Raumtemperatur unter Ausschluß von Luftsauerstoff gerührt. Die erhaltene Suspension wird eingeengt, mit 300 ml Methanol versetzt, 2 Stunden bei Raumtemperatur gerührt und abgesaugt. Das Filtrat wird vom Lösungsmittel befreit und der ölige Rückstand wird über Kieselgel mit Dichlormethan/Methanol 20/1 chromatographiert. Neben 5.4 g Diphenylamin werden 5.67 g (12.7 % d. Th.) $N^2$-Acetyl-6-O-diphenylcarbamoyl-7-[(1RS, 4SR)-4-hydroxy-cyclopent-2-en-1-yl]guanin vom Schmelzpunkt 146 - 149 °C erhalten. 1H NMR (270 MHz, $d_6$-DMSO), δ[ppm]: 10.70 (s, 1H), 8.38 (s, 1H), 7.53 - 7.41 (m, 8H), 7.37 - 7.29 (m, 2H), 6.21 (m, 1H), 6.03 (m, 1H), 5.47 (m, 1H), 5.21 (d, 1H), 4.74 (m, 1H), 2.93 (m, 1H), 2.21 (s, 3H), 1.80 (m, 1H).

1.14.

Verbindung der Formel III, wobei N = $N^2$-Acetylguanin-7-yl, n = 1 und $R^4$ = Acetoxy ist:
58.2 g (0.15 Mol) $N^2$-Acetyl-6-O-diphenylcarbamoyl-guanin werden in 125 ml Hexamethyldisilazan mit 0.5 g Ammoniumsulfat und 125 ml Xylol versetzt und 3 Stunden unter Rückfluß erhitzt. Die erhaltene Trimethylsilylverbindung wird wie oben beschrieben mit 5 Mol-% des in situ dargestellten Palladium-0-katalysators in 300 ml trockenem Tetrahydrofuran gelöst, tropfenweise mit der äquivalenten Menge Epoxycyclopenten versetzt und 3 Tage bei Raumtemperatur gerührt. Die erhaltene Lösung wird eingeengt, in Dichlormethan gelöst und mit katalytischen Mengen N,N-Dimethylaminopyridin und einem Überschuß Essigsäureanhydrid versetzt und 2 Stunden zum Rückfluß erhitzt. Das Reaktionsprodukt wird vom Lösungsmittel befreit und über Kieselgel mit Dichlormethan/Methanol 20/1 chromatographiert. Neben 16.6 g Diphenylamin werden 4.05 g (8.5% d. Th.) $N^2$-Acetyl-7-[(1RS,4SR)-4-acetoxy-cyclopent-2-en-1-yl]guanin vom Schmelzpunkt 218 - 222 °C erhalten. Das 1H NMR-Spektrum dieser Verbindung stimmt mit dem der N,O-Diacetylverbindung in der Beschreibung zum Beispiel 1.12.2., Methode c), überein.
13C NMR (67.93 MHz, $d_6$-DMSO), δ[ppm]: 173.28,169.91, 157.14, 152.64, 146.88, 141.34, 134.82, 134.04, 111.16, 76.80, 59.74, 38.88, 23.45, 20.73.

1.15.

Verbindung der Formel III, wobei N = 5-Benzyloxymethyl-1H-imidazo[4,5-d]pyridazin-4(5H)-on-1-yl, n = 1 und $R^4$ = Wasserstoff ist:
25.6 g 5-Benzyloxymethyl-1H-imidazo[4,5-d]pyridazin-4(5H)-on (0.1 Mol) werden in 140 ml trockenem Tetrahydrofuran wie oben beschrieben mit 5 Mol-% des in situ hergestellten Palladium-0-katalysators bei 0 °C langsam mit der äquivalenten Menge Epoxycyclopenten (gelöst in 45 ml trockenem Tetrahydrofuran) versetzt und anschließend 10 Stunden bei Raumtemperatur gerührt. Die erhaltene Reaktionslösung wird eingeengt und über neutralem Aluminiumoxid mit Dichlormethan/Methanol 20/1 chromatographiert. Man erhält 17.0 g (50.3 % d. Th.) 1-[(1RS, 4SR)-4-Hydroxy-cyclopent-2-en-1-yl]-5-benzyl-oxymethyl-1H-imidazo-[4,5-d]pyridazin-4(5H)-on mit dem Schmelzpunkt 122 - 123 °C. 1H NMR (270 MHz, $d_6$-DMSO), δ [ppm]: 8.48 (s, 1H), 8.36 (s, 1H), 7.32 (m, 5H), 6.22 (m, 1H), 6.08 (m, 1H), 6.00 (m, 1H), 5.59 (m, 2H), 5.26 (d, 1H), 4.73 (m, 1H), 4.69 (s, 2H), 2.97 (m, 1H), 1.69 (m, 1H).

1.16.

Verbindung der Formel III, wobei N = Cytosin-1-yl, n = 1 und $R^4$ = Wasserstoff ist:

a) Unter Argon-Schutzatmosphäre werden in einer Mischung aus je 220 ml trockenem Tetrahydrofuran und Dimethylsulfoxid 52.2 g (0.47 Mol) Cytosin mit 1 Mol-% des wie oben beschriebenen in situ hergestellten Palladium-0-katalysators durch langsame Zugabe mit 38.5 g (0.47 Mol) Epoxycyclopenten bei 0 °C zur Reaktion gebracht. Die Reaktionsmischung wird 90 Stunden bei Raumtemperatur gerührt, danach vom Lösungsmittel befreit und über Kieselgel mit Dichlormethan/Methanol 7/3 chromatographiert. Man erhält 71.8 g (79.2 % d. Th.) 1-[(1RS, 4SR)-4-Hydroxy-cyclopent-2-en-1-yl]cytosin mit dem Schmelzpunkt 241 - 242 °C. 1H-NMR (270 MHz, $d_6$-DMSO) $\delta$ [ppm]: 7.42 (d, 1H), 7.02 (s, 2H), 6.09 (m, 1H), 5.75 (m, 1H), 5.72 (d, 1H), 5.44 (m, 1H), 5.18 (d, 1H), 4.63 (m, 1H), 2.72 (m, 1H), 1.27 (m, 1H).

b) Setzt man trimethylsilyliertes Cytosin in diese Reaktion ein, so werden nach Alkoholyse des entstandenen silylierten Allylalkohols xx.x% d. Th. der unter 1.16.a. beschriebenen Verbindung isoliert.

1.17.

Verbindung der Formel III, wobei N = Uracil-1-yl, n = 1 und $R^4$ = Wasserstoff ist:

a) 5.79 g (0.03 Mol) der Verbindung des Beispiels 1.16. werden mit 15.3 g (0.18 Mol) Natriumnitrit in einer Mischung von 150 ml Wasser, 15 ml Eisessig und 30 ml 1N Salzsäure gelöst und 2 Tage bei Raumtemperatur gerührt. Die Reaktionsmischung wird eingeengt, mehrmals mit Aceton ausgekocht, die Acetonertrakte werden eingeengt und über Kieselgel mit Dichlormethan/Methanol 9/1 chromatographiert. Man erhält 3.65 g (62.7 % d. Th.) 1-[(1RS, 4SR)-4-Hydroxy-cyclopent-2-en-1-yl]-uracil als weißes Pulver mit dem Schmelzpunkt 163 - 164 °C. 1H NMR (270 MHz, $d_6$-DMSO) $\delta$ [ppm]:
11.23 (s, 1H), 7.45 (d, 1H), 6.13 (m, 1H), 5.79 (m, 1H), 5.64 (d, 1H), 5.39 (m, 1H), 5.24 (d, 1H), 4.63 (m, 1H), 2.73 (m, 1H), 1.36 (m, 1H).

b) Wird Uracil (21.5 g (0.192 Mol)) in Tetrahydrofuran/Dimethylsulfoxid mit 1 Mol-% des oben beschriebenen Palladium-0-katalysators mit der äquivalenten Menge Epoxycyclopenten in der üblichen Weise umgesetzt, so werden nach Chromatographie mit Dichlormethan/Methanol 9/1 an Aluminiumoxid neben dem $N^1$, $N^3$-Bisaddukt (1,3-Bis-[(1RS,4SR)-4-hydroxy-cyclopent-2-en-1-yl]uracil, Diastereomerengemisch, 21 g (39.6 % d. Th.); 1H NMR (270 MHz, $d_6$-DMSO), $\delta$[ppm]: 7.47 (d, 1H), 6.16 (m, 1H), 5.81 (m, 1H), 5.79 (s, 2H), 5.76 (md 1H), 5.63 (t, 1H), 5.41 (m, 1H), 5.26 (d, 1H), 4.76 (d, 1H), 4.61 (m, 1H), 2.74 (m, 1H), 2.50 (m, 1H), 1.94 (m, 1H), 1.38 (m, 1H)), welches als Öl anfällt, 0.3 g (0.8 % d. Th.) des $N^1$-Monoadduktes (Verbindung des Beispiels 1.17.) mit dem Schmelzpunkt 160 - 162 °C isoliert.

1.18.1. und 1.18.2.

Verbindungen der Formel III, wobei N = 5-Methyl-4-(1,2,4-triazol-1-yl)pyrimidin-2(1H)-on-1-yl, n = 1, $R^4$ = Wasserstoff (Beispiel 1.18.1.) und N = 5-Methyl-4-methoxy-pyrimidin-2(1H)-on-1-yl, n = 1 und $R^4$ = Wasserstoff (Beispiel 1.18.2.) ist: 3.54 g (0.020 Mol) 5-Methyl-4-(1,2,4-triazol-1-yl)pyrimidin-2(1H)-on (hergestellt durch Umsatz von Thymin mit 1,2,4-Triazol/$POCl_3$/Triethylamin) werden in einer Mischung aus 40 ml trockenem Tetrahydrofuran und 20 ml trockenem Dimethylsulfoxid mit 5 Mol% des wie oben in situ dargestellten Palladium-0-Katalysators bei 0 °C langsam mit der äquivalenten Menge Epoxycyclopenten versetzt, und die Reaktionsmischung wird 12 Stunden bei Raumtemperatur gerührt. Die erhaltene Suspension wird filtriert, das Filtrat wird eingeengt und der Rückstand wird an Kieselgel mit Dichlormethan/Methanol 9/1 chromatographiert. Man erhält 1.34 g (25.8 % d. Th.) 1-[(1RS, 4SR)-4-Hydroxy-cyclopent-2-en-1-yl]-5-methyl-4-(1,2,4-triazol-1-yl)-pyrimidin-2(1H)-on (Beispiel 1.18.1.) mit dem Schmelzpunkt 198 bis 204 °C. 1H NMR (270 MHz, $d_6$-DMSO), $\delta$[ppm]: 9.31 (s, 1H), 8.38 (s, 1H), 8.07 (s, 1H), 6.25 (m, 1H), 5.92 (m, 1H), 5.52 (m, 1H), 5.28 (d, 1H), 4.71 (m, 1H), 2.87 (m, 1H), 2.30 (s, 3H), 1.47 (m, 1H).

Als weiteres Produkt werden 0.59 g (13.2 % d. Th.) 1-[(1RS 4SR)-4-Hydroxy-cyclopent-2-en-1-yl]-5-methyl-4-methoxy-pyrimidin-2(1H)-on (Beispiel 1.18.2.) mit dem Schmelzpunkt 143 - 146 °C isoliert.
1H NMR (270 MHz, $d_6$-DMSO); $\delta$[ppm]: 7.58 (s, 1H), 6.16 (m, 1H), 5.81 (m, 1H), 5.48 (m, 1H), 5.21 (s, 1H), 4.56 (m, 1H), 3.86 (s, 3H), 2.79 (m, 1H), 1.89 (s, 3H), 1.33 (m, 1H).

1.19.

Verbindung der Formel III, wobei N = 4-Amino-5-Methyl-pyrimidin-2(1H)-on-1-yl, n = 1 und $R^4$ = Wasserstoff ist:

a) 0.4 g (1.54 mMol) der Verbindung aus Beispiel 1.18.1. werden in konzentriertem wäßrigen Ammoniak

suspendiert und 12 Stunden bei Raumtemperatur gerührt. Die Reaktionslösung wird vollständig eingeengt und über Kieselgel mit Dichlormethan/Methanol 5/1 chromatographiert. Man erhält 73 mg (22.9 % d. Th.) der Verbindung des Beispiels 1.20. mit einem Schmelzpunkt von 190 - 192 °C. Als zweite Fraktion werden 242 mg (75.9 % d. Th.) 4-Amino-5-methyl-1-[(1RS, 4SR)-4-hydroxy-cyclopent-2-en-1-yl]pyrimidin-2(1H)-on mit dem Schmelzpunkt 242 - 245 °C isoliert.

b) Zu einer Lösung des wie oben beschriebenen in situ hergestellten Palladium-0-katalysators (1.6 Mol%) in 200 ml trockenem Tetrahydrofuran/200 ml trockenem Dimethylsulfoxid werden bei 5 °C 50 g (0.4 Mol) 5-Methylcytosin zugegeben. In diese Suspension werden unter Rühren 49.2 g (0.6 Mol) Epoxycyclopenten, gelöst in 100 ml Tetrahydrofuran, zugetropft (1.5 Stunden). Die Mischung wird 3 Tage bei Raumtemperatur gerührt, anschließend eingeengt, mit 2N Natriumcarbonat-Lösung verrührt, filtriert und der Rückstand mit Wasser und Aceton gewaschen. Man erhält 20.2 g (24.4 % d. Th.) 4-Amino-5-Methyl-1-[(1RS,4SR)-4-hydroxy-cyclopent-2-en-1 yl]pyrimidin-2(1H)-on vom Schmelzpunkt 241 - 245 °C. Die Chromatographie (Kieselgel, Dichlormethan/Methanol 3/1) der neutralisierten Mutterlauge liefert neben 5-Methylcytosin (14.95 g, Schmelzpunkt 264 - 269 °C) weitere 9.87 g (11.9 % d. Th.) der Titelverbindung; Gesamtausbeute: 36.3 % d. Th. 1H NMR (270 MHz, $d_6$-DMSO) δ[ppm]: 7.23 (s, 1H), 7.12 (s, breit, 1H) 6.70 (s, breit, 1H), 6.09 (m, 1H), 5.74 (m, 1H), 5.45 (m, 1H), 5.17 (s, 1H), 4.61 (m, 1H), 2.71 (m, 1H), 1.81 (s, 3H), 1.27 (m, 1H).

1.20.

Verbindung der Formel III, wobei N = Thymin-1-yl, n = 1 und $R^4$ = Wasserstoff ist:

a) 0.4 g (1.54 mMol) der Verbindung des Beispiels 1.18.1. werden in 15 ml 2N Natronlauge suspendiert und 12 Stunden bei Raumtemperatur gerührt. Die erhaltene Lösung wird mit Eisessig neutralisiert, vollständig eingeengt und über Kieselgel mit Dichlormethan/Methanol 9/1 chromatographiert. Man erhält 316 mg (98.7 % d. Th.) 1-[(1RS, 4SR)-4-Hydroxy-cyclopent-2-en-1-yl]thymin mit dem Schmelzpunkt 191 - 192 °C.

b) 2.07 g (10 mMol) der Verbindung des Beispiels 1.19. werden in 50 ml Wasser suspendiert, mit 5.1 g (60 mMol) Natriumnitrit, 5 ml Eisessig und 10 ml 1N wäßriger Salzsäure versetzt und 10 Stunden bei Raumtemperatur gerührt. Die Reaktionsmischung wird eingeengt und mehrmals mit Aceton ausgekocht. Die Acetonextrakte werden eingeengt und über Kieselgel mit Dichlormethan/Methanol 9/1 chromatographiert. Man erhält 1.77 g (85.1 % d. Th.) 1-[(1RS, 4SR)-4-Hydroxy-cyclopent-2-en-1-yl]thymin vom Schmelzpunkt 191 - 192 °C. 1H NMR (270 MHz, $d_6$-DMSO), δ[ppm]: 11.23 (s, 1H), 7.28 (s, 1H), 6.13 (m, 1H), 5.79 (m, 1H), 5.38 (m, 1H), 5.21 (d, 1H), 4.62 (m, 1H), 2,72 (m, 1H), 1.76 (s, 3H), 1.36 (m, 1H).

1.21.1. und 1.21.2.

Verbindungen der Formel III, wobei N = 3-[(1RS, 4SR)-4-Hydroxy-cyclopent-2-en-1-yl]thymin-1-yl, n = 1 und $R^4$ = Wasserstoff (1.21.1.) und wobei N = Thymin-3-yl, n = 1 und $R^4$ = Wasserstoff (1.21.2.) ist:

In eine Lösung des wie oben beschriebenen in situ dargestellten Palladium-0-katalysators (0.62 Mol%) in 70 ml trockenem Tetrahydrofuran werden 80 ml trockenes Dimethylsulfoxid und 17.7 g (0.14 Mol) Thymin gegeben; anschließend werden bei 0 °C 11.5 g (0.14 Mol) Epoxycyclopenten, gelöst in 50 ml Tetrahydrofuran, langsam zugetropft (4 Stunden). Die Reaktionsmischung wird 20 Stunden bei Raumtemperatur gerührt, in 300 ml Ethanol gegeben, gerührt und filtriert. Das Filtrat wird eingeengt und über Aluminiumoxid mit Dichlormethan/Methanol 19/1 chromatographiert. Man erhält 15 g (36.9 % d. Th., bezogen auf eingesetztes Thymin) 1,3-Bis-[(1RS,4SR)-4-Hydroxy-cyclopent-2-en-1-yl]thymin (Diastereomerengemisch) als Öl (Verbindung 1.21.1.; 1H NMR (270 MHz, $d_6$-DMSO), δ[ppm]: 7.34 ("s", 1H), 6.15 (m, 1H), 5.83 - 5.75 (m, 3H), 5.67 (t, 1H), 5.41 (m, 1H), 5.22 (d, 1H), 4.76 (d, 1H), 4.62 (m, 2H), 2.76 (m, 1H), 2.50 (m, 1H), 1.93 (m, 1H) 1.80 (s, 3H), 1.38 (m, 1H)) und 0.9 g (3.1 % d. Th., bezogen auf eingesetztes Thymin) 3-[(1RS, 4SR)-4-Hydroxy-cyclopent-2-en-1-yl]thymin vom Schmelzpunkt 159 - 160 °C (Verbindung 1.21.2.). 1H NMR (270 MHz, $d_6$-DMSO), δ[ppm]: 10.85 (s, 1H), 7.29 (s, 1H), 5.78 (m, 2H), 5.62 (m, 1H), 4.77 (d, 1H), 4.58 (m, 1H), 2.50 (m, 1H), 1.89 (m, 1H), 1.76 (s, 3H).

1.22.

Verbindung der Formel III, wobei N = 3-Benzyloxymethyl-thymin-1-yl, n = 1 und $R^4$ = Wasserstoff ist: Bei 0 °C wird in 300 ml trockenem Tetrahydrofuran eine Lösung des oben beschriebenen Palladium-0-katalysators bereitet (5 Mol%). Zu dieser Lösung werden 24.6 g (0.1 Mol) 3-Benzyloxymethylthymin (hergestellt durch Reaktion von 1-Acetylthymin mit Benzylchlormethylether und Triethylamin in Dimethylformamid und nachfolgender Aminolyse mit wäßrigem Methylamin, Schmelzpunkt 120 °C) gegeben. Sodann wird eine Lösung von 12.3 g (0.15 Mol) Epoxycyclopenten in 50 ml Tetrahydrofuran zugetropft (45 Minuten). Die resultierende Mischung wird 48 Stunden bei Raumtemperatur gerührt, eingeengt und über Kieselgel mit Essigsäureethylester/Cyclohexan 2/1 chromatographiert. Neben 16.2 g 3-Benzyloxymethylthymin werden 2.59 g (23.1 % d. Th., bezogen auf umgesetztes 3-Benzyloxymethylthymin) 3-Benzyloxymethyl-1-[(1RS, 4SR)-4-hydroxy-cyclopent-2-en-1-yl]thymin mit dem Schmelzpunkt 101 - 104 °C erhalten. 1H NMR (270 MHz, $d_6$-DMSO),δ[ppm]: 7.31 (m, 6H); 6.15 (m, 1H), 5.79 (m, 1H), 5.43 (m, 1H), 5.36 (s, 2H), 5.23 (d, 1H), 4.64 (m, 1H), 4.60 (s, 2H), 2.75 (m, 1H), 1.80 (s, 3H), 1.37 (m, 1H).

1.23.

Verbindung der Formel III, wobei N = Pyridin-2(1H)-on-1-yl, n = 1 und $R^4$ = Wasserstoff ist:
Zu einer Lösung des wie oben dargestellten Palladium-0-katalysators (2 Mol% Palladium-II-acetat) in 200 ml trockenem Tetrahydrofuran werden bei 0 °C 21 g (0.22 Mol) 2-Pyridon zugegeben und danach über einen Zeitraum von 2 Stunden unter Rühren 20.1 g (0.245 Mol) Epoxycyclopenten, gelöst in 80 ml Tetrahydrofuran, zugetropft. Die sich bildende Lösung wird 2 Tage bei Raumtemperatur gerührt, danach eingeengt, in Dichlormethan gelöst, mit 1N Natriumcarbonatlösung ausgeschüttet und die organische Phase eingeengt. Der Rückstand wird über Kieselgel mit Essigsäureethylester/Methanol 9/1 chromatographiert. Man erhält 7.2 g (18.49 % d. Th.) 1-[(1RS, 4SR)-4-Hydroxy-cyclopent-2-en-1-yl]pyridin-2(1H)-on als zähes Öl. 1H NMR (60 MHz, $d_6$-DMSO), δ[ppm]: 7.65 - 7.25 (m, 2H), 6.55 - 6.08 (m, 3H), 5.95 - 5.63 (m, 2H), 5.26 (d, 1H), 4.73 (m, 1H), 2.85 (m, 1H), 1.32 (m, 1H).

1.24.

Verbindung der Formel III, wobei N = Pyridin-4(1H)-on-1-yl, n = 1 und $R^4$ = Wasserstoff ist:
Setzt man wie in Beispiel 1.23. beschrieben 4-Hydroxypyridin um, so erhält man nach Chromatographie (Aluminiumoxid, Dichlormethan/Methanol 9/1) 6.2 g (15.9 % d. Th.) 1-[(1RS, 4SR)-4-Hydroxy-cyclopent-2-en-1-yl]pyridin-4(1H)-on vom Schmelzpunkt 188 - 189 °C. 1H NMR (60 MHz, $d_6$-DMSO), δ [ppm]: 7.83 7.57 (m, 2H), 6.32 - 5.85 (m, 4H), 5.33 (d, 2H), 5.00 (m, 1H), 4.67 (m, 1H), 2.87 (m, 1H), 1.47 (m, 1H).

1.25.

Verbindung der Formel III, wobei N = 4,5-Dichlor-pyridazin-3(2H)-on-2-yl, n = 1 und $R^4$ = Wasserstoff ist:
Setzt man nach obiger Methode 32.8 g (0.2 Mol) 4,5-Dichlor-pyridazin-3(2H)-on um, so werden 19.5 g (39.47 % d. Th.) 4,5-Dichlor-2-[(1RS, 4SR)-4-hydroxy-cyclopent-2-en-1-yl]-pyridazin-3(2H)-on vom Schmelzpunkt 114 - 115 °C isoliert. 1H NMR (270 MHz, $d_6$-DMSO), δ[ppm]: 8.22 (s, 1H), 6.0 (m, 1H), 5.78 (m, 1H), 5.57 (m, 1H), 5.06 (d, 1H), 4.69 (m, 1H), 2.78 (m, 1H), 1.62 (m, 1H).

1.26.

Verbindung der Formel III, wobei N = N-Phthalimido, n = 1 und $R^4$ = Wasserstoff ist:
Setzt man nach obiger Methode in Tetrahydrofuran (1 Mol% Katalysator) 14.7 g (0.1 Mol) Phthalimid um, so werden 10.9 g (47.6 % d. Th.) (1RS, 4SR)-4-Hydroxy-1-phthalimido-cyclopent-2-en mit dem Schmelzpunkt 115- 116 °C isoliert. 1H NMR (60 MHz, $d_6$-DMSO), δ[ppm]: 7.88 (s, 4H), 5.93 (m, 2H), 5.25 - 4.40 (m, 4H), 2.58 (m, 1H), 2.0 (m, 1H).

1.27.

Verbindung der Formel III, wobei N = Adenin-9-yl, n = 2 und $R^4$ = Wasserstoff ist:
In 60 ml trockenem Tetrahydrofuran wird unter Argon bei 0 °C durch Zusammengeben von 179.6 mg Palladium-II-acetat, 2 ml Triisopropylphosphit und 1.06 ml einer 1.4 molaren Lösung von Butyllithium in n-Hexan der Palladium-0-katalysator hergestellt. Sodann werden 60 ml trockenes Dimethylsulfoxid und 17.32 g (0.128 Mol) Adenin zugefügt. Nach 15minütigem Rühren werden 12.3 g (0.128 Mol) 3,4-Epoxycyclohexen, gelöst in 40 ml trockenem Tetrahydrofuran, zugetropft (3 Stunden). Die Suspension wird 24 Stunden bei Raumtemperatur gerührt, in 650 ml Ethanol gegeben, gerührt und vom Niederschlag (1 g Adenin) abgesaugt. Das Filtrat wird eingeengt und über Aluminiumoxid mit Dichlormethan/Methanol 15/1 chromatographiert. Man erhält 7.96 g (36.9 % d. Th.) 9-[(1RS,4SR)-4-Hydroxy-cyclohex-2-en-1-yl]adenin mit dem

Schmelzpunkt 181 bis 183°C. 1H NMR (270 MHz, $d_6$-DMSO) δ[ppm]: 8.15 (s, 1H), 8.04 (s, 1H), 7.22 (s, 2H), 6.09 (m, 1H), 5.84 (m, 1H), 5.06 (m, 1H), 4.93 (d, 1H), 4.09 (m, 1H), 1.99 (m, 2H), 1.81 (m, 1H), 1.56 (m, 1H).

1.28.1.

Verbindung der Formel IV, wobei N = Cytosin-1-yl und $R^4$ = Wasserstoff ist:
In einer Mischung aus je 120 ml trockenem Tetrahydrofuran und Dimethylsulfoxid werden 27.75 g (0.25 Mol) Cytosin mit 2 Mol% des in situ erzeugten Palladium-0-Katalysators und 0.275 Mol 3,4-Epoxycyclohexen wie in dem vorangegangenen Beispiel beschrieben umgesetzt. Das Rohprodukt wird abgesaugt, mit Tetrahydrofuran und Ethanol gewaschen und liefert nach Kristallisation aus Ethanol 29 g (56 % d. Th.) 1-[-(1RS, 2SR)-2-Hydroxy-cyclohex-5-en-1-yl]cytosin mit dem Schmelzpunkt 264°C (Zers.). 1H NMR (270 MHz, $d_6$-DMSO), δ[ppm]: 7.27 (d, 1H), 6.94 (d, 2H), 5.96 (m, 1H), 5.63 (d, 1H), 5.35 (m, 1H), 5.14 (m, 1H), 4.89 (d, 1H), 3.93 (s, breit, 1H), 2.21 (m, 1H), 1.94 (m, 1H), 1.74 (m, 2H).

1.28.2.

Verbindung der Formel III, wobei N = Cytosin-1-yl, n = 2 und $R^4$ = Wasserstoff ist:
Aus den Mutterlaugen, die bei der Aufarbeitung der Verbindung 1.28.1. anfallen, werden durch Chromatographie an Kieselgel mit Dichlormethan/Methanol 3/1 4.3 g (6.6 % d. Th.) 1-[(1RS, 4SR)-4-Hydroxy-cyclohex-2-en-1-yl]cytosin mit dem Schmelzpunkt 224 - 226°C gewonnen. 1H NMR (270 MHz, $d_6$-DMSO), δ[ppm]: 7.44 (d, 1H), 7.00 (s, 2H), 6.04 (m, 1H), 5.70 (d, 1H), 5.55 (m, 1H), 4.97 (m, 1H), 4.86 (d, 1H), 4.00 (m, 1H), 1.70 (m, 3H), 1.50 (m, 1H).

1.29.1.

Verbindung der Formel IV, wobei N = Uracil-1-yl und $R^4$ = Wasserstoff ist:
4.14 g (0.02 Mol) der Verbindung des Beispiels 1.28.1. werden mit 8.28 g (0.12 Mol) Natriumnitrit in 130 ml Wasser, 13 ml Eisessig und 20 ml 1N wäßriger Salzsäure gelöst und 2 Tage bei Raumtemperatur gerührt. Die Reaktionslösung wird eingeengt, mit wenig eiskaltem Wasser angerührt, abgesaugt und mit wenig kaltem Wasser gewaschen. Man erhält 3.6 g (86.5 % d. Th.) 1-[(1RS,2SR)-2-Hydroxy-cyclohex-5-en-1-yl]-uracil mit dem Schmelzpunkt 203°C. 1H NMR (270 MHz, $d_6$-DMSO), δ[ppm]: 11.18 (s, 1H), 7.27 (d, 1H), 6.04 (m, 1H), 5.50 (m, 1H), 5.39 (m, 1H), 5.07 (d, 1H), 5.03 (m, 1H), 3.96 (m, 1H), 2.21 (m, 1H), 1.95 (m, 1H), 1.76 (m, 2H).

1.29.2.

Verbindung der Formel III, wobei N = Uracil-1-yl, n = 2 und $R^4$ = Wasserstoff ist:
1.55 g (7.5 mMol) der Verbindung des Beispiels 1.28.2. werden mit 3.1 g (45 mMol) Natriumnitrit in 50 ml Wasser mit 5 ml Eisessig und 7.5 ml 1N wäßriger Salzsäure gelöst und 48 Stunden bei Raumtemperatur gerührt. Die erhaltene Lösung wird eingeengt und über Kieselgel mit Dichlormethan/Methanol 5/1 chromatographiert. Man erhält 1.35 g (86.5% d. Th.) 1-[(1RS, 4SR)-4-Hydroxy-cyclohex-2-en-1-yl]uracil mit dem Schmelzpunkt 161 - 162°C. 1H NMR (60 MHz, $d_6$-DMSO), δ[ppm]: 11.33 (s, 1H), 7.53 (d, 1H), 6.13 (m, 1H), 5.8 - 5.5 (m, 2H), 4.92 (m, 2H), 4.03 (m, 1H), 1.75 (m, 4H).

1.30.1.

Verbindung der Formel IV, wobei N = N-Phthalimido und $R^4$ = Wasserstoff ist:
Werden 19.11 g (0.13 Mol) Phthalimid mit 0.6 Mol% des wie oben beschriebenen in situ hergestellten Palladium-0-katalysators und einem Äquivalent 3,4-Epoxycyclohexen in Tetrahydrofuran erst bei 0°C und dann für 4 Stunden bei Rückflußtemperatur umgesetzt, so werden nach chromatographischer Reinigung an Kieselgel mit Essigsäureethylester/Cyclohexan 3/2 1.45 g (4.6 % d. Th.) (1RS,2SR)-2-Hydroxy-1-phthalimido-cyclohex-5-en mit dem Schmelzpunkt 112 bis 114°C gewonnen. 1H NMR (270 MHz, $d_6$-DMSO), δ [ppm]: 7.83 (m, 4H), 5.90 (m, 1H), 5.63 (m, 1H), 4.84 (d, 1H), 4.75 (m, 1H), 3.90 (m, 1H), 2.27 (m, 1H), 2.1 - 1.65 (m, 3H).

1.30.2

Verbindung der Formel III, wobei N = N-Phthalimido, n = 2 und $R^4$ = Wasserstoff ist:
Die weitergeführte chromatographische Reinigung des Reaktionsansatzes 1.30.1. liefert 9.62 g (33 % d. Th.) (1RS, 4SR)-4-Hydroxy-1-phthalimido-cyclohex-2-en mit dem Schmelzpunkt 126 - 129 °C. 1H NMR (270 MHz, $d_6$-DMSO), δ [ppm]: 7.85 (m, 4H), 5.83 (m, 1H), 5.72 (m, 1H) 4.76 (d, 1H), 4.60 (m, 1H), 4.04 (m, 1H), 2.24 (m, 1H), 1.90 - 1.55 (m, 3H).

1.31.

Verbindung der Formel III, wobei N = Guanin-9-yl, n = 2 und $R^4$ = Wasserstoff ist:
7.56 g (50 mMol) Guanin werden mit 2 Mol% des wie oben in situ hergestellten Palladium-0-katalysators mit einem Äquivalent 3,4-Epoxycyclohexen in Tetrahydrofuran erst bei 0 °C und dann 8 Stunden unter Rückflußtemperatur umgesetzt und danach in Methanol gegeben. Die Reaktionsmischung wird eingeengt, in 2-Propanol aufgenommen und abgesaugt. Der Rückstand wird zuerst mit Aktivkohle aus Ethanol und danach aus Wasser umkristallisiert. Man erhält 3.1 g (25.1 % d. Th.) 9-[(1RS, 4SR)-4-Hydroxy-cyclohex-2-en-1-yl]guanin mit dem Schmelzpunkt >300 °C. 1H NMR (270 MHz, $d_6$-DMSO), δ[ppm]: 10.57 (s, 1H), 7.60 (s, 1H) 6.46 (s, 2H), 6.05 (m, 1H), 5.77 (m, 1H) 4.91 (d, 1H) 4.79 (m, 1H), 4.06 (m, 1H), 1.95 - 1.75 (m, 3H), 1.53 (m, 1H).

1.32.

1-[(4RS)-4-Hydroxy-cyclohex-1-en-1-yl)thymin:
6.3 g (50 mMol) Thymin werden mit 2 Mol% des wie oben beschriebenen in situ hergestellten Palladium-0-Katalysators mit einem Äquivalent 3,4-Epoxycyclohexen in Tetrahydrofuran erst bei 0 °C und dann 4 Stunden bei Rückflußtemperatur behandelt. Die Reaktionsmischung wird mit Methanol versetzt, die Suspension wird filtriert, das Filtrat wird eingeengt und über Kieselgel mit Dichlormethan/Methanol 20/1 chromatographiert. Man erhält 1.8 g (16.2 % d. Th.) 1-[(4RS)-4-Hydroxy-cyclohex-1-en-1-yl]thymin mit dem Schmelzpunkt 223 °C. 1H NMR (270 MHz, $d_6$-DMSO), δ[ppm]: 11.23 (s, 1H), 7.69 (s, 1H), 4.59 (m, 1H), 2.84 (t, 1H), 2.42 - 1.81 (m, 6H), 1.79 (s, 3H), 1.61 (m, 1H).

Acylderivate:

Die Acylderivate werden durch Reaktion des entsprechenden Säureanhydrids (z. B. Essigsäureanhydrid) mit dem jeweiligen Alkohol unter Katalyse mit N,N-Dimethylaminopyridin in einem Lösungsmittel, wie z. B. Dichlormethan, oder durch Reaktion mit einem Säureanhydrid in Pyridin hergestellt.

2.1.1.

Verbindung der Formel V, wobei N = $N^6$-Acetyl-adenin-9-yl, n = 1 und $R^5$ = Acetyl ist:
Farblose Kristalle, Fp.: 153 - 154 °C

2.1.2.

Verbindung der Formel V, wobei N = $N^6$-Diacetyl-adenin-9-yl, n = 1 und $R^5$ = Acetyl ist:
Öl, 1H NMR (270 MHz, $d_6$-DMSO), δ[ppm]: 8.98 (s, 1H), 8.57 (s, 1H), 6.38 (m, 1H), 6.31 (m, 1H), 5.75 (m, 1H), 5.67 (m, 1H), 3.10 (m, 1H), 2.25 (s, 6H), 2.07 (m, 1H), 2.03 (s, 3H).

2.1.3.

Verbindung der Formel V, wobei N = Adenin-9-yl, n = 1 und $R^5$ = Aceryl ist:
Farbloses Pulver, Fp.: 130 - 133 °C

2.1.4.

Verbindung der Formel V, wobei N = $N^6$-Acetyl-adenin-9-yl, n = 1 und $R^5$ = Wasserstoff ist:
Farblose Kristalle, Fp.: 134 - 136 °C, 1H NMR (60 MHz, $d_6$-DMSO), δ[ppm]: 10.6 (s, 1H), 8.67 (s, 1H), 8.40 (s, 1H), 6.13 (m, 2H), 5.57 (m, 1H), 5.35 (d, 1H), 4.73 (m, 1H), 2.95 (m, 1H), 2.27 (s, 3H), 1.73 (m, 1H).

2.2.

Verbindung der Formel V, wobei N = Hypoxanthin-9-yl, n = 1 und $R^5$ = Acetyl ist:
Farblose Kristalle, Fp.: 215 - 218 °C

2.3.

Verbindung der Formel V, wobei N = 6-Chlorpurin-9-yl, n = 1 und $R^5$ = Acetyl ist:
Farblose Kristalle, Fp.: 114 - 115 °C

2.4.1.

Verbindung der Formel V, wobei N = 2-Acetamido-6-chlor-purin-9-yl, n = 1 und $R^5$ = Acetyl ist:
Farblose Kristalle, Fp.: 180 - 181 °C

2.4.2.

Verbindung der Formel V, wobei N = 2-Acetamido-6-chlor-purin-9-yl, n = 1 und $R^5$ = Wasserstoff ist:
Blaßgelbe Kristalle, Fp.: 191 - 193 °C, 1H NMR (270 MHz, $d_6$-DMSO), δ[ppm]: 10.83 (s, 1H), 8.43 (s, 1H), 6.21 (m, 1H), 6.03 (m, 1H), 5.20 (d, 1H), 4.75 (m, 1H), 2.93 (m, 1H), 2.21 (s, 3H), 1.82 (m, 1H).

2.4.3.

Verbindung der Formel V, wobei N = 2-Amino-6-chlor-purin-9-yl, n = 1 und $R^5$ = Acetyl ist:
Hellgelbe Kristalle, Fp.: 183 - 185 °C, 1H NMR (60 MHz, $d_6$-DMSO), δ[ppm]: 8.0 (s, 1H), 6.95 (s, 2H), 6.33 (m, 2H), 5.80 - 5.32 (m, 2H), 3.28 - 2.73 (m, 1H), 2.07 (s, 1H), 1.93 (m, 1H).

2.5.

Verbindung der Formel V, wobei N = 2-Acetamido-6-ethoxy-purin-9-yl, n = 1 und $R^5$ = Wasserstoff ist:
Blaßgelbe Kristalle, Fp.: 126 - 128 °C, 1H NMR (270 MHz, $d_6$-DMSO), δ[ppm]: 10.32 (s, 1H), 8.13 (s, 1H), 6.19 (m, 1H), 6.0 (m, 1H), 5.42 (m, 1H), 5.21 (d, 1H), 4.73 (m, 1H), 4.57 (q, 2H), 2.90 (m, 1H), 2.23 (s, 3H), 1.77 (m, 1H), 1.40 (t, 3H).

2.6.1.

Verbindung der Formel V, wobei N = $N^2$-Acetyl-guanin-9-yl, n = 1 und $R^5$ = Acetyl ist:
Farblose Kristalle, Fp.: 247 °C

2.6.2.

Verbindung der Formel V, wobei N = Guanin-9-yl, n = 1 und $R^5$ = Acetyl ist:
Farblose Kristalle, Fp.: 243 °C, 1H NMR (270 MHz, $d_6$-DMSO), δ[ppm]: 10.58 (s, 1H), 7.51 (s, 1H), 6.45 (s, 2H), 6.23 (m, 2H), 5.62 (m, 1H), 5.30 (m, 1H), 2.97 (m, 1H), 2.02 (s, 3H), 1.80 (m, 1H).

2.7.

Verbindung der Formel V, wobei N = 5-Benzyloxymethyl-1H-imidazo[4,5-d]pyridazin-4(5H)-on-1-yl, n = 1 und $R^5$ = Acetyl ist:
Blaßrote Kristalle, Fp.: 105 °C

2.8.1.

Verbindung der Formel V, wobei N = $N^4$-Acetyl-cytosin-1-yl, n = 1 und $R^5$ = Acetyl ist:
Farblose Kristalle, Fp.: 196 °C

2.8.2.

Verbindung der Formel V, wobei N = Cytosin-1-yl, n = 1 und $R^5$ = Acetyl ist:

Blaßgelbe Kristalle, Fp.: 48 - 52°C (Zers.), 1H NMR (270 MHz, $d_6$-DMSO), δ[ppm]: 7.41 (d, 1H), 7.02 (s, 2H), 6.09 (m, 1H), 5.75 (m, 1H), 5.72 (d, 1H), 5.45 (m, 1H), 4.63 (m, 1H), 2.72 (m, 1H), 1.91 (s, 3H), 1.27 (m, 1H).

2.9.1. Verbindung der Formel V, wobei N = Uracil-1-yl, n = 1 und $R^5$ = Acetyl ist:

Farblose Kristalle, Fp.: 131 - 136°C

2.9.2.

Verbindung der Formel V, wobei N = 3-(4-Acetoxy-cyclo-pent-2-en-1-yl)uracil-1-yl, n = 1 und $R^5$ = Acetyl ist:
Farbloses Pulver, Fp.: 148°C

2.10.

Verbindung der Formel V, wobei N = Thymin-1-yl, n = 1 und $R^5$ = Acetyl ist:
Farblose Kristalle, Fp.: 149 - 150°C; 1H NMR (270 MHz, $d_6$-DMSO), δ[ppm]: 11.27 s, 1H), 7.12 (s, 1H), 6.20 (m, 1H), 6.08 (m, 1H), 5.54 (m, 1H), 5.45 (m, 1H), 2.84 (m, 1H), 2.03 (s, 3H), 1.78 (s, 3H), 1.57 (m, 1H).

2.11.

Verbindung der Formel V, wobei N = 3-(4-Acetoxy-cyclo-pent-2-en-1-yl)thymin-1-yl, n = 1 und $R^5$ = Acetyl ist:
Öl, 1H NMR (270 MHz, $d_6$-DMSO), δ[ppm]: 7.15 (s, 1H), 6.21 (m, 1H), 6.09 (m, 1H), 6.04 (m, 1H), 5.8 - 5.67 (m, 2H), 5.61 - 5.42 (m, 3H), 2.88 (m, 1H), 2.64 (m, 1H), 2.11 (m, 1H), 2.03 (s, 6H), 1.81 (s, 3H), 1.6 (m, 1H).

2.12.

Verbindung der Formel V, wobei N = 4,5-Dichlor-pyridazin-3(2H)-on-2-yl, n = 1 und $R^5$ = Acetyl ist:
Farblose Kristalle, Fp.: 108°C

2.13.

Verbindung der Formel V, wobei N = Pyridin-4(1H)-on-1-yl, n = 1 und $R^5$ = Acetyl ist:
Farblose Kristalle, Fp.: 109 - 110°C

2.14.

Verbindung der Formel V, wobei N = Pyridin-2(1H)-on-1-yl, n = 1 und $R^5$ = Acetyl ist:
Farbloses Öl; 1H NMR (60 MHz, $d_6$-DMSO), δ[ppm]: 7.37 m, 2H), 6.60 - 6.08 (m, 4H), 5.95 - 5.53 (m, 2H), 2.92 m, 1H), 2.03 (s, 3H), 1.47 (m, 1H).

2.15.

Verbindung der Formel V, wobei N = N-Phthalimido, n = 1 und $R^5$ = Acetyl ist:
Farblose Kristalle, Fp.: 85 - 86°C

2.16.

Verbindung der Formel V, wobei N = Guanin-9-yl, n = 2 und $R^5$ = Acetyl ist:
Farblose Kristalle, Fp.: >290°C, 1H NMR (270 MHz, $d_6$-DMSO), δ[ppm]: 10.81 (s, 1H), 7.81 (s, 1H), 6.18 - 6.03 (m, 4H), 5.23 (m, 2H), 2.16 - 1.83 (m, 7H), 1.74 - 1.60 (m, 1H).

2.17.

Verbindung der Formel VI, wobei N = $N^4$-Acetyl-cytosin-1-yl, n = 2 und $R^5$ = Acetyl ist:
Farblose Kristalle, Fp.: 211°C

2.18.

Verbindung der Formel V, wobei N = N-Phthalimido, n = 2 und $R^5$ = Acetyl ist:
Farblose Kristalle, Fp.: 153 - 155 °C

Triolderivate:

Zur Herstellung der Verbindungen der Formeln VIIa bzw. Ia und und VIIIa bzw. IIa werden 50 mMol der ungesättigten Verbindungen der Formeln V bzw. III oder VI bzw. IV mit 100 - 150 mMol N-Methylmorpholin-N-oxid-Hydrat in einer Mischung aus 200 ml Wasser und 100 ml Tetrahydrofuran gelöst bzw. suspendiert (wobei zur Erzielung einer homogenen Lösung bei Bedarf Aceton zugegeben werden kann), mit 0.5 - 2.0 Mol% einer 1 %igen Lösung von Osmium-tetroxid in Wasser versetzt und 5 Stunden bis 6 Tage bei Raumtemperatur gerührt. Danach wird die Reaktionsmischung mit 100 - 200 mMol Natriumhydrogensulfit versetzt, eingeengt und über Kieselgel chromatographisch gereinigt.

3.1.1.

Verbindung der Formel Ia, wobei N = Adenin-9-yl, n = 1 und $R^1$, $R^2$ und $R^3$ = Wasserstoff ist:
Die cis-Hydroxylierung der Verbindung des Beispiels 2.1.1., durchgeführt nach der oben beschriebenen Methode, liefert in 52.7%iger Ausbeute $N^6$-Acetyl-9-[(1RS,2SR, 3SR,4SR)-4-acetoxy-2,3-dihydroxy-cyclopent-1-yl]adenin (Verbindung der Formel VIIa, wobei N = $N^6$-Acetyl-adenin-9-yl, n = 1 und $R^5$ = Acetyl ist) mit dem Schmelzpunkt 193 - 195 °C. Diese Verbindung wird in Pyridin suspendiert, mit konzentriertem wäßrigen Ammoniak versetzt und 6 Stunden bei 60 °C gerührt. Nach Kristallisation aus Ethanol/Wasser 9/1 erhält man in 86 %iger Ausbeute 9-[(1RS, 2SR, 3RS, 4SR)-2,3,4-Trihydroxy-cyclopent-1-yl]adenin mit dem Schmelzpunkt 282 °C (Hemihydrat). 1H NMR (270 MHz, $d_6$-DMSO), $\delta$[ppm]: 8.17 (s, 1H), 8.13 (s, 1H), 7.23 (s, 2H), 5.36 (d, 1H), 5.01 (d, 1H), 4.87 (d, 1H), 4.7 (m, 1H), 4.53 (m, 1H), 3.92 (m, 1H, 3.78 (m, 1H), 2.62 (m, 1H), 1.83 (m, 1H).

3.1.2.

Verbindung der Formel Ia, wobei N = $N^6$-Acetyl-adenin-9yl, n = 1, $R^1$-$R^2$ = Dimethylmethylen und $R^3$ = Acetyl ist:
Die Umsetzung der Verbindung der Formel VIIa des Beispiels 3.1.1. mit 2,2-Dimethoxypropan in Aceton mit p-Toluolsulfonsäure als Katalysator liefert $N^6$-Acetyl-9-[(1RS, 2SR, 3SR, 4SR)-4-acetoxy-2,3-dihydroxy-2,3-O-isopropyliden-cyclopent-1-yl]adenin mit dem Schmelzpunkt 82 - 83 °C (Hemihydrat, 54.4% d. Th.). 1H NMR (270 MHz, $d_6$-DMSO), $\delta$[ppm]: 10.71 (s, 1H), 8.68 (s, 1H), 8.59 (s, 1H), 5.22 (m, 1H), 5.1 - 5.0 (m, 2H), 4.82 (d, 1H), 2.72 (m, 1H), 2.44 (m, 1H), 2.28 (s, 3H), 1.93 (s, 3H), 1.50 (s, 3H), 1.29 (s, 3H).

3.1.3.

Verbindung der Formel Ia, wobei N = Adenin-9-yl, n = 1, $R^1$-$R^2$ = Dimethylmethylen und $R^3$ = Acetyl ist:
Aus obiger Reaktion wird neben der Verbindung des Beispiels 3.1.2. in einer Ausbeute von 23.3% 9-[-(1RS,2SR,3SR,4SR)-4-Acetoxy-2,3-dihydroxy-2,3-O-isopropyliden-cyclopent-1-yl]adenin mit dem Schmelzpunkt 229 - 230 °C gewonnen. 1H NMR (270 MHz, $d_6$-DMSO), $\delta$[ppm]: 8.25 (s, 1H), 8.19 (s, 1H), 7.4 (s, 2H), 5.18 (m, 1H), 5.04 (m, 1H), 4.89 (m, 1H), 4.80 (d, 1H), 2.68 (m, 1H), 2.40 (m, 1H), 1.96 (s, 3H), 1.49 (s, 3H), 1.28 (s, 3H).

3.1.4.

Verbindung der Formel Ia, wobei N = Adenin-9-yl, n = 1, $R^1$-$R^2$ = Dimethylmethylen und $R^3$ = Wasserstoff ist:
Die Ammonolyse der Verbindung des Beispiels 3.1.3. liefert 9-[(1RS,2SR,3RS, 4SR)2,3,4-Trihydroxy-2,3-O-isopropyliden-cyclopent-1-yl]adenin (68.1 % d. Th., Fp.: 124 °C (Zers.)). 1H NMR (400 MHz, $d_6$-DMSO), $\delta$-[ppm]: 8.25 (s, 1H), 8.14 (s, 1H), 7.18 (s, 2H), 5.51 (d, 1H), 4.94 (m, 1H), 4.82 (m, 1H), 4.55 (d, 1H), 4.17 (m, 1H), 2.52 (m, 1H), 2.19 (m, 1H), 1.43 (s, 3H), 1.23 (s, 3H).

3.2.

Verbindung der Formel Ia, wobei N = Hypoxanthin-9-yl, n = 1 und $R^1$, $R^2$ und $R^3$ = Wasserstoff ist: Die cis-Hydroxylierung der Verbindung des Beispiels 2.2., durchgeführt nach der oben beschriebenen Methode, liefert in 98.6 %iger Ausbeute 9-[(1RS,2SR,3SR,4SR)-4-Acetoxy-2,3-dihydroxy-cyclopent-1-yl]-hypoxanthin (Verbindung der Formel VIIa, wobei N = Hypoxanthin-9-yl, n = 1 und $R^5$ = Acetyl ist) mit dem Schmelzpunkt 240 - 242°C als Hemihydrat. Diese Verbindung wird in Pyridin suspendiert, mit konzentriertem wäßrigen Ammoniak versetzt und 6 Stunden bei 60°C gerührt. Man erhält in 97.6 %iger Ausbeute 9-[(1RS,2SR,3RS,4SR)-2,3,4-Trihydroxy-cyclopent-1-yl]hypoxanthin (Hydrat) mit dem Schmelzpunkt 198 bis 201°C.

3.3

Verbindung der Formel Ia, wobei N = $N^6$-Methyl-adenin-9-yl, n = 1 und $R^1$, $R^2$ und $R^3$ = Wasserstoff ist:
Die cis-Hydroxylierung der Verbindung des Beispiels 2.3. - durchgeführt wie oben beschrieben - liefert in 61.4 %iger Ausbeute 6-Chlor-9-[(1RS,2SR,3SR,4SR)-4-acetoxy-2,3-dihydroxy-cyclopent-1-yl]purin (Verbindung der Formel VIIa, wobei N = 6-Chlorpurin-9-yl, n = 1 und $R^5$ = Acetyl ist) mit dem Schmelzpunkt 103 - 104°C. Diese Verbindung wird in Methanol gelöst, mit 40 %igem wäßrigem Methylamin versetzt und eine Stunde bei Rückflußtemperatur gerührt. Nach Chromatographie (Kieselgel, Dichlormethan/Methanol 2/1) erhält man in 78 %iger Ausbeute $N^6$-Methyl-9-[(1RS, 2SR, 3RS, 4SR)-trihydroxy-cyclopent-1-yl]adenin mit dem Schmelzpunkt 180°C (Zers.). 1H NMR (270 MHz, $d_6$-DMSO), $\delta$-[ppm]: 8.2 (s, 1H), 8.14 (s, 1H), 7.67 (s, 1H), 5.35 (d, 1H), 4.98 (d, 1H), 4.84 (d, 1H), 4.68 (m, 1H), 4.51 (m, 1H), 3.90 (m, 1H), 3.74 (m, 1H), 2.98 (s, 3H), 2.61 (m, 1H), 1.83 (m, 1H).

3.4.

Verbindung der Formel Ia, wobei N = 2-Aminopurin-9-yl, n = 1 und $R^1$, $R^2$, und $R^3$ = Wasserstoff ist: Die cis-Hydroxylierung der Verbindung des Beispiels 2.4.1. - durchgeführt wie oben beschrieben - liefert in 41 %iger Ausbeute 2-Acetamido-6-Chlor-9-[(1RS,2SR,3SR,4SR)-4-acetoxy-2,3-dihydroxy-cyclopent-1-yl]-purin (Verbindung der Formel VIIa, wobei N = 2-Acetamido-6-Chlor-purin-9-yl, n = 1 und $R^5$ = Acetyl ist) mit dem Schmelzpunkt 198°C. Diese Verbindung wird in Methanol unter Zugabe von Triethylamin und Palladium auf Kohle suspendiert und bei Normaldruck hydriert. Die Hydrogenolyse ergibt in 85 %iger Ausbeute 2-Acetamido-9-[(1RS,2SR,3SR,4SR)-4-acetoxy-2,3-dihydroxy-cyclopent-1-yl]purin (Verbindung der Formel VIIa, wobei N = 2-Acetamidopurin-9-yl, n = 1 und $R^5$ = Acetyl ist) mit dem Schmelzpunkt 222 - 223°C. Diese Diacetylverbindung wird in Methanol unter Zugabe von wäßrigem Methylamin gelöst und eine Stunde unter Rückfluß gekocht. Man erhält in 71 %iger Ausbeute 2-Amino-9-[(1RS,2SR,3RS,4SR)-2,3,4-trihydroxycyclopent-1-yl]purin mit dem Schmelzpunkt 153 - 155°C. 1H NMR (270 MHz, $d_6$-DMSO), $\delta$[ppm]: 8.57 (s, 1H), 8.12 (s, 1H), 6.47 (s, 1H), 5.15 (d, 1H), 4.99 (d, 1H), 4.81 (d, 1H), 4.66 (m, 1H), 4.45 (m, 1H), 3.93 (m, 1H), 3.76 (m, 1H), 2.57 (m, 1H), 1.72 (m, 1H).

3.5.

Verbindung der Formel Ia, wobei N = Guanin-9-yl, n = 1 und $R^1$, $R^2$ und $R^3$ = Wasserstoff ist: Die cis-Hydroxylierung der Verbindung des Beispiels 2.6.1. - durchgeführt wie oben beschrieben - liefert in 88 %iger Ausbeute $N^2$-Acetyl-9-[(1RS,2SR,3SR,4SR)-4-acetoxy-2,3-dihydroxy-cyclopent-1-yl]guanin (Verbindung der Formel VIIa, wobei N = $N^2$-Acetyl-guanin-9-yl, n = 1 und $R^5$ = Acetyl ist) mit dem Schmelzpunkt 234°C. Diese Verbindung wird wie oben beschrieben mit Methanol und wäßriger Methylaminlösung behandelt und liefert nach Kristallisation aus Ethanol/Wasser in 69 %iger Ausbeute 9-[-(1RS,2SR,3RS,4SR)-2,3,4-Trihydroxy-cyclopent-1-yl]guanin (Hemihydrat) mit dem Schmelzpunkt 295°C. 1H NMR (270 MHz, $d_6$-DMSO), $\delta$[ppm]: 10.50 (s, 1H), 7.74 (s, 1H), 6.36 (s, 2H), 5.14 (d, 1H), 4.95 (d, 1H), 4.77 (d, 1H), 4.51 (m, 1H), 4.37(m, 1H), 3.86 (m, 1H), 3.72 (m, 1H), 2.53 (m, 1H), 1.61 (m, 1H).

3.6.

Verbindung der Formel Ia, wobei N = 1H-Imidazo[4,5-d]pyridazin-4(5H)-on-1-yl, n = 1 und $R^1$, $R^2$ und $R^3$ = Wasserstoff ist:
Die cis-Hydroxylierung der Verbindung des Beispiels 1.15. - durchgeführt wie oben beschrieben - liefert in 87 %iger Ausbeute 5-Benzyloxymethyl-1-[(1RS,2SR,3RS,4SR)-2,3,4-trihydroxy-cyclopent-1-yl]imidazo[4,5-d]pyridazin-4(5H)-on (Verbindung der Formel VIIa, wobei N = 5-Benzyloxymethyl-1H-imidazo[4,5-d]-

pyridazin-4(5H)-on-1-yl, n = 1 und $R^5$ = Wasserstoff ist) als glasigen Schaum. Diese Verbindung wird in Methanol mit Palladium auf Kohle als Katalysator hydriert und liefert in 79 %iger Ausbeute 1-[-(1RS,2SR,3RS,4SR)-2,3,4-Trihydroxy-cyclo-pent-1-yl]imidazo[4,5-d]pyridazin-4(5H)-on als Schaum. Die Titelverbindung kann auch gewonnen werden, wenn man die Verbindung des Beispiels 2.7. nach der oben beschriebenen Methode der cis-Hydroxylierung unterwirft und danach die Benzyloxymethylschutzgruppe hydrogenolytisch und die Acetylschutzgruppe hydrolytisch abspaltet.

3.7.

Verbindung der Formel Ia, wobei N = Cytosin-1-yl, n = 1 und $R^1$, $R^2$ und $R^3$ = Wasserstoff ist:
Die cis-Hydroxylierung der Verbindung des Beispiels 2.8.1. - durchgeführt wie oben beschrieben - liefert in 49 %iger Ausbeute $N^4$-Acetyl-1-[(1RS,2SR,3SR,4SR)-4-acetoxy-2,3-dihydroxy-cyclopent-1-yl]cytosin (Verbindung der Formel VIIa, wobei N = $N^4$-Acetyl-cytosin-1-yl, n = 1 und $R^5$ = Acetyl ist) mit dem Schmelzpunkt 98 bis 100 °C. Diese Verbindung wird in Pyridin mit konzentriertem wäßrigen Ammoniak bei 60 °C behandelt und liefert in 84 %iger Ausbeute 1-[(1RS,2SR,3SR,4SR)-4-acetoxy-2,3-dihydroxy-cyclopent-1-yl]cytosin (Verbindung der Formel VIIa, wobei N = Cytosin-1-yl, n = 1 und $R^5$ = Acetyl ist) mit dem Schmelzpunkt 121 - 124 °C. Die Reaktion dieser Verbindung mit wäßrigem Methylamin in Methanol bei Rückflußtemperatur liefert in 78 %iger Ausbeute 1-[(1RS,2SR,3RS,4SR)-2,3,4-Trihydroxy-cyclopent-1-yl]cytosin mit dem Schmelzpunkt 155 °C (Zers.). 1H NMR (270 MHz, $d_6$-DMSO), $\delta$[ppm]: 7.64 (d, 1H), 7.19 (s, breit, 2H), 5.76 (d, 1H), 5.20 (s, breit, 1H), 4.76 (s, breit, 2H), 4.59 (m, 1H), 4.19 (m, 1H), 3.78 (m, 1H), 3.63 (m, 1H), 2.40 (m, 1H), 1.38 (m, 1H). Die Titelverbindung kann auch durch cis-Hydroxylierung der Verbindung des Beispiels 1.16. gewonnen werden.

3.8.

Verbindung der Formel Ia, wobei N = Uracil-1-yl, n = 1 und $R^1$, $R^2$ und $R^3$ = Wasserstoff ist:
Die cis-Hydroxylierung der Verbindung des Beispiels 1.17. - dürchgeführt wie oben beschrieben - liefert in 79 %iger Ausbeute 1-[(1RS,2SR,3RS,4SR)-2,3,4-Trihydroxycyclopent-1-yl]uracil mit dem Schmelzpunkt 229 - 230 °C. 1H NMR (270 MHz, $d_6$-DMSO), $\delta$[ppm]: 11.18 (s, 1H), 7.66 (d, 1H), 5.65 (d, 1H), 5.11 (d, 1H), 4.92 (d, 1H), 4.78 (d, 1H), 4.73 (m, 1H), 4.11 (m, 1H), 3.82 (m, 1H), 3.65 (m, 1H), 2.42 (m, 1H), 1.34 (m, 1H).

3.9.

Verbindung der Formel Ia, wobei N = Thymin-1-yl, n = 1 und $R^1$, $R^2$ und $R^3$ = Wasserstoff ist:
Die cis-Hydroxylierung der Verbindung des Beispiels 2.10. - durchgeführt wie oben beschrieben - liefert in 78 %iger Ausbeute 1-[(1RS,2SR,3SR,4SR)-4-Acetoxy-2,3-dihydroxy-cyclopent-1-yl]thymin (Verbindung der Formel VIIa, wobei N = Thymin-1-yl, n = 1 und $R^5$ = Acetoxy ist) mit dem Schmelzpunkt 58 - 61 °C. 1H NMR (270 MHz, $d_6$-DMSO), $\delta$[ppm]: 11.30 (s, 1H), 7.49 (d, 1H), 5.15 (d, 1H), 5.08 (d, 1H), 4.74 (m, 1H), 4.63 (m, 1H), 4.16 (m, 1H), 3.85 (m, 1H), 2.43 (m, 1H), 2.05 (s, 3H), 1.80 (s, 3H), 1.58 (m, 1H). Diese Verbindung wird in Pyridin mit wäßriger Methylaminlösung behandelt und liefert in 84 % Ausbeute 1-[-(1RS,2SR,3RS,4SR]-2,3,4-Trihydroxy-cyclo-pent-1-yl]thymin mit dem Schmelzpunkt 201 - 202 °C. 1H NMR (270 MHz, $d_6$-DMSO), $\delta$[ppm]: 11.18 (s, 1H), 7.54 (s, 1H), 5.10 (d, 1H), 4.87 (d, 1H), 4.77 (d, 1H), 4.70 (m, 1H), 4.14 (m, 1H), 3.81 (m, 1H), 3.66 (m, 1H), 2.40 (m, 1H), 1.79 (s, 3H), 1.34 (m, 1H).

3.10.1.

Verbindung der Formel Ia, wobei N = 4,5-Dichlor-pyridazin-3(2H)-on-2-yl, n = 1 und $R^1$, $R^2$ und $R^3$ = Wasserstoff ist:
Die cis-Hydroxylierung der Verbindung des Beispiels 1.25. - durchgeführt wie oben beschrieben - liefert in 59 %iger Ausbeute 4,5-Dichlor-2-[(1RS,2SR,3RS,4SR)-2,3,4-trihydroxy-cyclopent-1-yl]pyridazin-3(2H)-on mit dem Schmelzpunkt 163 °C. 1H NMR (270 MHz, $d_6$-DMSO), $\delta$[ppm]: 8.26 (s, 1H), 5.17 (m, 1H), 4.95 (d, 1H), 4.82 (d, 1H), 4.77 (d, 1H), 4.29 (m, 1H), 3.84 (m, 1H), 3.67 (m, 1H), 2.41 (m, 1H), 1.49 (m, 1H).

3.10.2.

Verbindung der Formel Ia, wobei N = 4-Chlor-5-Methoxy-pyridazin-3(2H)-on-2-yl, n = 1 und $R^1$, $R^2$ und $R^3$ = Wasserstoff ist:
Die cis-Hydroxylierung der Verbindung des Beispiels 2.12. - durchgeführt wie oben beschrieben - liefert in

49 %iger Ausbeute 4,5-Dichlor-2-[(1RS,2SR,3SR,4SR)-4-acetoxy-2,3-dihydroxy-cyclopent-1-yl]pyridazin-3-(2H)-on (Verbindung der Formel VIIa, wobei N = 4,5-Dichlor-pyridazin-3(2H)-on-2-yl, n = 1 und $R^5$ = Acetyl ist) mit dem Schmelzpunkt 119 - 122 °C. Diese Verbindung wird mit 0.1 Äquivalenten KCN in Methanol gelöst und 12 Stunden bei Raumtemperatur gerührt. Sodann wird durch Zugabe eines Anionenaustauschers (OH-Form) alkalisch und nach 5 Minuten durch Zugabe eines Kationenaustauschers ($H^+$-Form) neutralisiert. Man filtriert vom Ionenaustauscher ab, engt das Filtrat ein und chromatographiert den Rückstand über Kieselgel (Essigsäureethylester/Methanol 20/1). Neben 38 % der Verbindung des Beispiels 3.10.1. werden 27 % 4-Chlor-5-Methoxy-2-[(1RS,2SR,3RS,4SR)-2,3,4-trihydroxy-cyclopent-1-yl]pyridazin-3-(2H)-on mit dem Schmelzpunkt 166 - 168 °C isoliert. 1H NMR (270 MHz, $d_6$-DMSO), δ[ppm]: 8.32 (s, 1H), 5.19 (m, 1H), 4.94 (d, 1H), 4.75 (d, 1H), 4.73 (d, 1H), 4.30 (m, 1H), 4.07 (s, 3H), 3.84 (m, 1H), 3.68 (m, 1H), 2.87 (m, 1H), 1.44 (m, 1H).

3.11.

Verbindung der Formel Ia, wobei N = Pyridin-4(1H)-on-1-yl, n = 1 und $R^1$, $R^2$ und $R^3$ = Wasserstoff ist:
Die cis-Hydroxylierung der Verbindung des Beispiels 2.13. - durchgeführt wie oben beschrieben - liefert in 81 %iger Ausbeute 1-[(1RS,2SR,3SR,4SR)-4-Acetoxy-2,3-dihydroxy-cyclopent-1-yl]pyridin-4(1H)-on (Verbindung der Formel VIIa, wobei N = Pyridin-4(1H)-on-1-yl, n = 1 und $R^5$ = Acetyl ist) mit dem Schmelzpunkt 191 bis 196 °C. Diese Verbindung wird in Methanol unter Zugabe von wäßriger Methylaminlösung gelöst und 90 Minuten zum Rückfluß erhitzt. Die Aufarbeitung liefert in 79 %iger Ausbeute 1-[-(1RS,2SR,3RS,4SR)-2,3,4-Trihydroxy-cyclopent-1-yl]pyridin-4(1H)-on mit dem Schmelzpunkt 186 - 188 °C. 1H NMR (270 MHz, $d_6$-DMSO), δ[ppm]: 7.70 (d, 2H), 6.01 (d, 2H), 5.24 (d, 1H), 5.06 (d, 1H), 4.95 (d, 1H), 4.14 (m, 2H), 3.86 (m, 1H), 3.68 (m, 1H), 2.60 (m, 1H), 1.53 (m, 1H).

3.12.

Verbindung der Formel Ia, wobei N = Pyridin-2(1H)-on-1-yl, n = 1 und $R^1$, $R^2$ und $R^3$ = Wasserstoff ist:
Die cis-Hydroxylierung der Verbindung des Beispiels 2.14. - durchgeführt wie oben beschrieben - liefert in 81 %iger Ausbeute 1-[(1RS,2SR,3SR,4SR)-4-Acetoxy-2,3-dihydroxy-cyclopent-1-yl]pyridin-2(1H)-on (Verbindung der Formel VIIa, wobei N = Pyridin-2(1H)-on-1-yl, n = 1 und $R^5$ = Acetyl ist) mit dem Schmelzpunkt 133 bis 135 °C. Diese Verbindung wird in Methanol unter Zusatz von wäßrigem Methylamin gelöst und 90 Minuten bei Rückflußtemperatur gehalten. Man erhält in 80 %iger Ausbeute 1-[-(1RS,2SR,3RS,4SR)-2,3,4-Trihydroxy-cyclopent-1-yl]pyridin-2(1H)-on mit dem Schmelzpunkt 138 - 140 °C. 1H NMR (270 MHz, $d_6$-DMSO), δ[ppm]: 7.72 (m, 1H), 7.37 (m, 1H), 6.31 (m, 2H), 5.19 (d, 1H), 5.03 (m, 1H), 4.77 (m, 2H), 4.28 (m, 1H), 3.85 (m, 1H), 3.72 (m, 1H), 2.53 (m, 1H), 1.37 (m, 1H).

3.13.

Verbindung der Formel Ia, wobei N = N-Phthalimido, n = 1 und $R^1$, $R^2$ und $R^3$ = Wasserstoff ist:
Die cis-Hydroxylierung der Verbindung des Beispiels 1.26. - durchgeführt wie oben beschrieben - liefert in 72 %iger Ausbeute (1RS,2SR,3RS,4SR)-2,3,4-Trihydroxy-1-phthalimido-cyclopentan mit dem Schmelzpunkt 175 - 176 °C. 1H NMR (60 MHz, $d_6$-DMSO), δ[ppm]: 7.9 (s, 4H), 4.9 (m, 2H), 4.6 (m, 3H), 3.73 (m, 2H), 2.05 (m, 2H).

3.14.

Verbindung der Formel Ia, wobei N = Cytosin-1-yl, n = 2 und $R^1$, $R^2$ und $R^3$ = Wasserstoff ist:
Die cis-Hydroxylierung der Verbindung des Beispiels 1.28.2. - durchgeführt wie oben beschrieben - mit nachfolgender Peracylierung (Essigsäureanhydrid/Pyridin/4-Dimethylaminopyridin/20 Stunden/Raumtemperatur) des entstandenen Triols liefert in 83 %iger Ausbeute $N^4$-Acetyl-1[-(1RS,2SR,3RS,4SR)-2,3,4-Triacetoxycyclohex-1-yl]cytosin (Verbindung der Formel Ia, wobei N = Cytosin-1-yl, n = 2 und $R^1$ - $R^3$ = Acetyl ist) mit dem Schmelzpunkt 226 °C. 1H NMR (270 MHz, $d_6$-DMSO), δ[ppm]: 10.83 (s, 1H), 8.17 (d, 1H), 7.16 (d, 1H), 5.50 (m, 1H), 5.30 (m, 1H), 4.90 (m, 1H), 4.83 (m, 1H), 2.15 (s, 3H), 2.14 (s, 3H), 2.09 (s, 3H), 1.94 - 1.74 (m, 4H), 1.83 (s, 3H). Diese Verbindung wird in Methanol gelöst, mit 40 %igem wäßrigen Methylamin versetzt und 2 Stunden unter Rückfluß gekocht. Man erhält in 94 %iger Ausbeute 1-[(1RS,2SR,3RS,4SR)-2,3,4-Trihydroxy-cyclohex-1-yl]cytosin mit dem Schmelzpunkt 271 °C

(Zers.). 1H NMR (270 MHz, $d_6$-DMSO), δ[ppm]: 7.48 (d, 1H), 6.86 (s, breit, 2H), 5.56 (d, 1H), 4.79 (d, 1H), 4.74 (d, 1H), 4.59 (m, 1H), 4.27 (d, 1H), 3.86 (m, 1H), 3.74 (m, 2H), 1.73 (m, 2H), 1.44 (m, 2H).

3.15.

Verbindung der Formel IIa, wobei N = Cytosin-1-yl und $R^1$, $R^2$ und $R^3$ = Wasserstoff ist:
Die cis-Hydroxylierung der Verbindung des Beispiels 2.17. - durchgeführt wie oben beschrieben - liefert in 65 %iger Ausbeute $N^4$-Acetyl-1-[(1RS,2SR,3RS,6RS)-6-acetoxy-2,3-dihydroxy-cyclohex-1-yl]cytosin (Verbindung der Formel VIIIa, wobei N = $N^4$-Acetyl-cytosin-1-yl und $R^5$ = Acetyl ist) mit dem Schmelzpunkt 249°C. Diese Verbindung wird in Methanol gelöst, mit 40 %igem wäßrigen Methylamin zum Rückfluß erhitzt und liefert nach der Aufarbeitung des Reaktionsgemisches in 97 %iger Ausbeute 1-[-(1RS,2SR,3RS,6RS).2,3,6-Trihydroxy-cyclohex-1-yl]cytosin mit dem Schmelzpunkt 304°C (Zers.). 1H NMR (270 MHz, $d_6$-DMSO), δ[ppm]: 7.45 (d, 1H), 6.80 (s, breit, 2h), 5.58 (d, 1H), 4.79 (s, breit, 1H), 4.63 (m, 1H), 4.54 (d, 1H), 4.42 (d, 1H), 3.87 (m, 3H), 1.80 (m, 2H), 1.47 (m, 2H).

3.16.

Verbindung der Formel IIa, wobei N = Uracil-1-yl und $R^1$, $R^2$ und $R^3$ = Wasserstoff ist:
Die cis-Hydroxylierung der Verbindung des Beispiels 1.29.1. - durchgeführt wie oben beschrieben - liefert in 72 %iger Ausbeute 1-[(1RS,2SR,3RS,6RS)-2,3,6-Trihydroxy-cyclohex-1-yl]uracil mit dem Schmelzpunkt 246°C (umkrist. aus Ethanol). 1H NMR (270 MHz, $d_6$-DMSO), δ[ppm]: 11.12 (s, 1H), 7.51 (d, 1H), 5.49 (d, 1H), 4.67 (m, 2H), 4.56 (m, 1H), 3.88 (m, 3H), 3.18 (d, 1H), 1.70 (m, 2H), 1.48 (m, 2H).

Gesättigte Verbindungen:

Pneumocystis carinii - Versuchsbeschreibung und Ergebnisse
Spraque-Dawley Ratten, 200 - 220 g, wurden in Gruppen zu 10 Tieren zusammengesetzt und mit Dexamethason 1,5 mg/ml; Ofloxacin 0,2 mg/Ratte über das Trinkwasser behandelt, um den Ausbruch der durch Pneumocystis carinii bedingten Lungenentzündung zu induzieren.

Nach 7 Wochen wurde die Dexamethason-Behandlung eingestellt und mit der Präparate-Behandlung begonnen.

Über zwei Wochen wurde jeweils montags, mittwochs und freitags wie folgt behandelt:

Tabelle I

| Gruppe | Verbindung | Dosis |
|---|---|---|
| 1. | Beispiel 3.10.1 | 20 mg/kg oral |
| 2. | Pentamidin | 20 mg/kg i.m. |
| 3. | Pentamidin | 40 mg/kg i.m. |

Nach den zwei Behandlungswochen wurde bei den Tieren in Nembutalnarkose eine Bronchiallavage durchgeführt.

Die gewonnen Bronchialsuspensionen wurden mit dem Pneumocystis carinii-Testkit der Fa. Progen Biotechnik GmbH, Heidelberg, Deutschland aufgearbeitet und ausgewertet.

Die Ergebnisse sind in Tabelle II zusammengefaßt.

Tabelle II

| Gruppe | Reduktion (%) |
|---|---|
| 1. | 22,9 |
| 2. | 37,7 |
| 3. | 16,9 |

In vitro - Versuche und Ergebnisse (antivirale Wirkung)
Die antivirale Wirksamkeit der erfindigsgemäßen Verbindungen wurde in in vitro Versuchen geprüft. Dazu wurden die erfindungsgemäßen Verbindungen in verschiedenen Verdünnungen zu Zellkulturen von HeLa-Zellen in Mikrotiterplatten gegeben. Nach 3 Stunden wurden die Kulturen mit Virus infiziert. HeLa-Zellen wurden mit Vaccinia-Virus infiziert. 48 - 72 Stunden nach der Infektion wurde der Therapieerfolg anhand des cytopathischen Effektes mikroskopisch und nach Neutralrotaufnahme (Farbtest nach Finter) photometrisch bestimmt (Finter, N.B., in "Interferones" (N.B. Finter et al.), North Holland Publishing Co., Amsterdam (1966)). Die minimale Konzentration, bei der etwa die Hälfte der Zellen keinen cytopathogenen Effekt zeigt, wird als minimale Hemmkonzentration (MHK) betrachtet. Ergebnisse sind in Tabelle III zusammengefaßt. (DTM = Dosis tolerata maxima)

Tabelle III

| Verbindung aus Beispiel | Vaccinia |
|---|---|
| 3.1.1 | MHK: 0.05 µg/ml<br>DTM: >400 µg/ml |
| 3.3 | MHK: 44.4 µg/ml<br>DTM: >400 µg/ml |

**Patentansprüche**

1. Verbindungen der Formeln I und II,

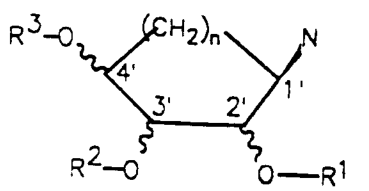

Formel I

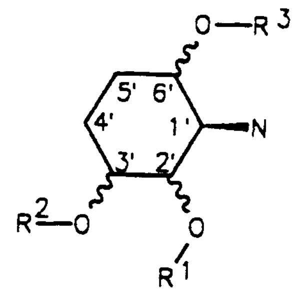

Formel II

in denen N ein über Stickstoff an C-1'gebundener mono- oder bicyclischer Heterocyclus mit mindestens einem Stickstoffatom ist, der unabhängig voneinander mit bis zu drei Amino-, Hydroxy-, Mercapto- und/oder Halogengruppen substituiert sein kann und wobei die Amino-, Hydroxy- und/oder Mercaptogruppen unabhängig voneinander bis zu zweifach mit C1-C6-Alkyl, C1-C6-Acyl-, Benzyl-, Trityl-, Benzyloxymethyl-, Amidingruppen und/oder Phenyl ggfs. seinerseits substituiert mit Halogen, C1-C6-Alkyl, C1-C6-Alkoxy, Amino, C1-C6-Alkylamino oder C1-C12-Dialkylamino substituiert sein können und wobei NH-Funktionen des Heterocyclus mit C1-C6-Acyl-, Benzyl- oder Benzyloxymethylgruppen substituiert sein können, und

n 1 - 4 ist,

und

$R^1$, $R^2$ und $R^3$ unabhängig voneinander Wasserstoff, C1-C8-Alkyl, C3-C8-Alkenyl, C3-C8-Alkinyl, C3-C8-Cycloalkyl, C3-C8-Cycloalkenyl, Benzyl, Trityl, Benzyloxymethyl oder C1-C8-Acyl sind,

oder

$R^1$ und $R^2$ Teil eines 1,3-Dioxa-pentanringes sind, in dessen 2-Position ein oder zwei Wasserstoffatome, unabhängig voneinander mit C1-C8-Alkyl, C2-C16-Dialkyl, C3-C8-Cycloalkyl, C3-C8-Cycloalkenyl, Phenyl, Diphenyl, C1-C8-Alkoxy, C2-C16-Dialkoxy, Dimethylamino, Sauerstoff oder Schwefel substituiert sein können, oder Teil eines cyclischen Sulfits, cyclischen Sulfats, cyclischen Phosphits oder cyclischen Phosphats sind, oder

beide Wasserstoffatome ersetzt sind durch die Gruppe - $CH_2$-$CH_2$-$CH_2$-N($CH_3$)-

und

$R^3$ wie oben angegeben ist,

sowie deren physiologisch verträgliche Salze und offensichtliche chemische Äquivalente, wobei die Verbindung der Formel C

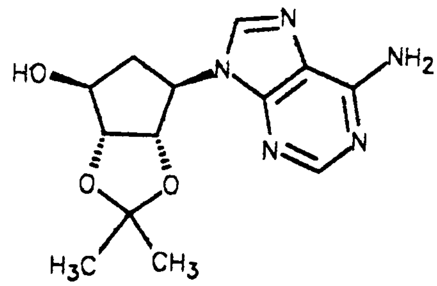

C

ausgenommen ist.

2. Verbindungen der Formeln Ia und IIa

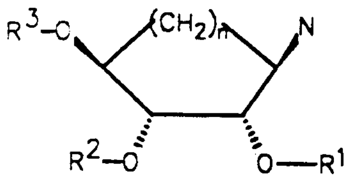

Formel Ia

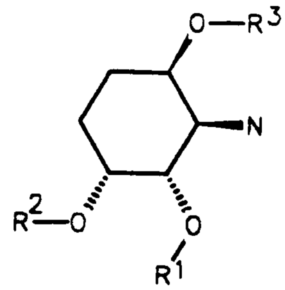

Formel IIa

in denen die Substituenten N, $R^1$, $R^2$ und $R^3$ sowie n wie in Anspruch 1 angegeben sind.

3. Verbindungen der Formeln Ia und IIa, gemäß Anspruch 2, in denen N ein über Stickstoff an C-1' gebundenes Purin, Pyrimidin, 8-Azapurin, 1-Deazapurin, 3-Deazapurin, Benzimidazol, Imidazo[4,5-d]-pyridazin, Pyridazin, Pyridin, Triazol, Imidazol oder Imid ist, das unabhängig voneinander mit bis zu drei Amino-, Hydroxy-, Mercapto- und/oder Halogengruppen substituiert sein kann und wobei die Amino-, Hydroxy- und/oder Mercaptogruppen unabhängig voneinander bis zu zweifach mit C1-C6-Alkyl-, C1-C6-Acyl-, Benzyl-, Trityl-, Benzyloxymethyl-, Amidingruppen und/oder Phenyl ggfs. seinerseits substituiert mit Halogen, C1-C6-Alkyl, C1-C6-Alkoxy, Amino, C1-C6-Alkylamino oder C1-C12-Dialkylamino substituiert sein können und wobei NH-Funktionen des Heterocyclus mit C1-C3-Acyl-, Benzyl- oder Benzyloxymethylgruppen substituiert sein können,

und

n 1 - 3 ist,

und

$R^1$, $R^2$ und $R^3$ unabhängig voneinander Wasserstoff, C1-C5-Alkyl, C3-C5-Alkenyl, C3-C5-Alkinyl, C3-C5-Cycloalkyl, C3-C5-Cycloalkenyl, Benzyl, Trityl, Benzyloxymethyl oder C1-C7-Acyl sind,

oder

$R^1$ und $R^2$ Teil eines 1,3-Dioxa-pentanringes sind, in dessen 2-Position ein oder zwei Wasserstoffatome, unabhängig voneinander mit C1-C8-Alkyl, C2-C8-Dialkyl, C3-C8-Cycloalkyl, C3-C8-Cycloalkenyl, Phenyl, Diphenyl, C1-C4-Alkoxy, C2-C8-Dialkoxy, Dimethylamino, Sauerstoff oder Schwefel substituiert sein können, oder Teil eines cyclischen Sulfits, cyclischen Sulfats, cyclischen Phosphits oder cyclischen Phosphats sind, oder

beide Wasserstoffatome ersetzt sind durch die Gruppe $-CH_2-CH_2-CH_2-N(CH_3)-$

und

$R^3$ wie oben angegeben ist, sowie deren physiologisch verträgliche Salze und offensichtliche chemische Äquivalente.

4. Verbindungen der Formeln Ia und IIa gemäß einem oder mehreren der Ansprüche 1 bis 3, in denen N ein über Stickstoff an C-1' gebundenes Purin, Pyrimidin, 3-Deazapurin, Imidazo[4,5-d]pyridazin, Pyridazin, Pyridin, Imidazol oder Imid ist, das unabhängig voneinander mit bis zu drei Amino-, Hydroxy-, Mercapto- und/oder Halogengruppen substituiert sein kann und wobei die Amino-, Hydroxy- und/oder Mercaptogruppen unabhängig voneinander bis zu zweifach mit C1-C3-Alkyl-, C1-C3-Acyl-, Benzyl-, Trityl-, Amidingruppen und/oder Phenyl ggfs. seinerseits substituiert mit Halogen, C1-C3-Alkyl, C1-C3-Alkoxy, Amino, C1-C3-Alkylamino oder C1-C6-Dialkylamino substituiert sein können,

und

n 1 oder 2 ist

und

$R^1$, $R^2$ und $R^3$ unabhängig voneinander Wasserstoff, C1-C3-Alkyl, Allyl, Propargyl, Cyclopentyl, Benzyl, Trityl, Benzyloxymethyl oder C1-C4-Acyl sind,

oder

$R^1$ und $R^2$ Teil eines 1,3-Dioxa-pentanringes sind, in dessen 2-Position ein oder zwei Wasserstoffatome, unabhängig voneinander mit C1-C3-Alkyl, C2-C6-Dialkyl, C4-C6-Cycloalkyl, C4-C5-Cycloalkenyl, Phenyl, Diphenyl, C1-C3-Alkoxy, C2-C6-Dialkoxy, Sauerstoff oder Schwefel substituiert sein können, oder Teil eines cyclischen Sulfits, cyclischen Sulfats, cyclischen Phosphits oder cyclischen Phosphats sind,

und

$R^3$ wie oben angegeben ist, sowie deren physiologisch verträgliche Salze und offensichtliche chemische Äquivalente.

5. Verbindungen der Formel Ia und IIa gemäß einem oder mehreren der Ansprüche 1 bis 4, in denen N ein über Stickstoff an C-1' gebundenes Purin, Pyrimidin, 3-Deazapurin, Pyridazin oder Imidazol ist, das unabhängig voneinander mit bis zu drei Amino-, Hydroxy-, Mercapto- und/oder Halogengruppen substituiert ist und wobei die Amino-, Hydroxy- und/oder Mercaptogruppen unabhängig voneinander bis zu zweifach mit C1-C3-Alkyl-, C1-C3-Acyl-, Benzylgruppen und/oder Phenyl ggfs. seinerseits substituiert mit Chlor, C1-C3-Alkyl, C1-C3-Alkoxy oder Amino substituiert sein können,

und

n 2 ist

und

$R^1$, $R^2$ und $R^3$ unabhängig voneinander Wasserstoff, C1-C3 -Alkyl, Allyl, Propargyl, Cyclopentyl, Benzyl, Trityl, Benzyloxymethyl oder C1-C4-Acyl sind, oder

$R^1$ und $R^2$ Teil eines 1,3-Dioxa-pentanringes sind, in dessen 2-Position ein oder zwei Wasserstoffatome, unabhängig voneinander mit C1-C3-Alkyl, Cyclopentyl, Phenyl, Diphenyl, C1-C3-Alkoxy, Sauerstoff oder Schwefel substituiert sein können,

und

$R^3$ wie oben angegeben ist, sowie deren physiologisch verträgliche Salze und offensichtliche chemische Äquivalente.

6. Verbindungen der Formeln Ia und IIa, gemäß einem oder mehreren der Ansprüche 1 - 5 wobei die Verbindung der Formel C nicht außgenommen ist, sowie deren physiologisch verträgliche Salze und offensichtliche chemische Äquivalente zur Prophylaxe oder Behandlung von Erkrankungen, die durch Viren oder einzellige Parasiten verursacht werden.

7. Verfahren zur Herstellung von Verbindungen der Formeln III und IV,

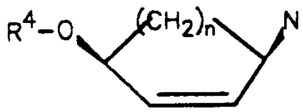

Formel III

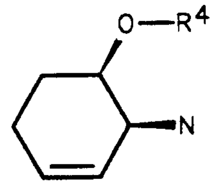

Formel IV

dadurch gekennzeichnet, daß ein Äquivalent eines stickstoffhaltigen Heterocyclus, der wie in den Ansprüchen 1-5 definiert ist, mit mindestens einer freien NH-Funktion oder mindestens einer silylierten, bevorzugt trimethylsilylierten, oder mindestens einer acylierten, bevorzugt acetylierten, N-Funktion im Heterocyclus, und wobei weitere NH-Funktionen im und Amino-, Hydroxy- und Mercapto-Funktionen am Heterocyclus mit N-Funktion im Heterocyclus, und wobei weitere NH-Funktionen im und Amino-, Hydroxy- und Mercapto-Funktionen am Heterocyclus mit Trimethylsilyl, Acyl, Benzyl, Benzyloxymethyl, Dimethylaminomethyliden oder N-Methyl-2-pyrrolidinyliden substituiert sein können, mit 0.001 bis 0.5 Äquivalenten, bevorzugt 0.005 - 0.1 Äquivalenten eines Palladium-(0)-Katalysators wie Tetrakis-(triphenylphosphin)palladium(0) oder Tetrakis(triisopropylphospit)palladium(0), bevorzugt Tetrakis-(triisopropylphosphit)palladium(0), welcher in situ aus Palladium-II-acetat, dem jeweiligen Phosphin oder Phosphit und Butyllithium hergestellt werden kann, und mit 1 Äquivalent 1,2-Epoxycycloalk-3-en, bevorzugt Epoxycyclo-penten oder 1,2-Epoxy-cyclohex-3-en in einem Lösungsmittel wie Tetrahydrofuran, Toluol,Dimethylsulfoxid, Dimethylformamid oder N-Methylpyrrolidon-(2) oder Gemischen davon, bevorzugt in Tetrahydrofuran, Dimethylsulfoxid oder Dimethylformamid oder Gemischen davon bei 0 ° C bis 110 ° C, bevorzugt anfangs bei Raumtemperatur und später bei 80 - 90 ° C unter einer Schutzgasatmosphäre, beispielsweise Argon, während 2 bis 24 Stunden, bevorzugt während 2 - 10 Stunden, zu Verbindungen der Formeln III oder/und IV umgesetzt wird, wobei $R^4$ Wasserstoff ist, wenn in der obigen Reaktion ein stickstoffhaltiger Heterocyclus mit mindestens einer nicht modifizierten NH-Funktion eingesetzt wird oder $R^4$ Trialkylsilyl, bevorzugt Trimethylsilyl, ist, wenn in der obigen Reaktion ein stickstoffhaltiger Heterocyclus, dessen NH-Funktion trialkylsilyl-, bevorzugt trimethylsilylmodifiziert, war eingesetzt wird oder $R^4$ Acyl, bevorzugt Acetyl, ist, wenn in der obigen Reaktion ein stickstoffhaltiger Heterocyclus, dessen NH-Funktion acyl-, bevorzugt acetyl-modifiziert, war eingesetzt wird.

**8.** Verfahren zur Herstellung von Verbindungen der Formeln I und II, gemäß den Ansprüchen 1-5, dadurch gekennzeichnet, daß Verbindungen der Formeln V oder VI,

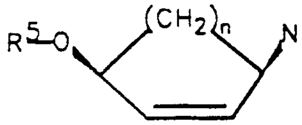

Formel V

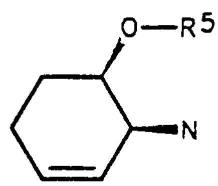

Formel VI

wobei $R^5$ = $R^4$, Alkyl, Benzyl, Trityl oder Benzyloxymethyl ist, und N ein stickstoffhaltiger Heterocyclus wie in den Ansprüchen 1-5 definiert, ist, und wobei eventuell vorhandene Amino-, Hydroxy- und Mercapto-Gruppen am Heterocyclus und weitere NH-Gruppen im Heterocyclus mit Trimethylsilyl, C1-C6-Alkyl, C1-C6-Acyl, Benzyl, Trityl, Benzyloxymethyl, Dimethylaminomethyliden oder N-Methyl-2-pyrrolidinyliden substituiert sein können, mit 1 - 5 Äquivalenten, bevorzugt 3 Äquivalenten, N-Methylmorpholin-N-oxid und einer katalytischen Menge Osmiumtetroxid in Tetrahydrofuran, Aceton oder Wasser oder Gemischen davon umgesetzt wird, wobei Verbindungen der Formeln VII und VIII, gebildet werden

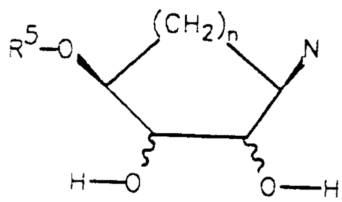

Formel VII

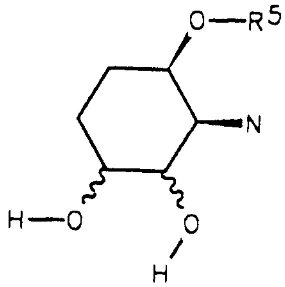

Formel VIII

und daß Verbindungen der Formeln VII und VIII, ihrerseits durch Standardoperationen in Verbindungen der Formeln I und II, wobei die Substituenten N, $R^1$, $R^2$ und $R^3$ die in den Ansprüchen 1-5 genannten Bedeutungen haben, umgewandelt werden.

**9.** Verbindungen der Formeln III, IV, V, VI, VII, VIIa, VIII und VIIIa,

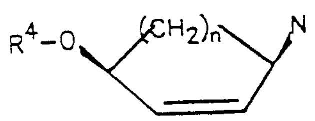

Formel III

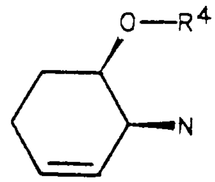

Formel IV

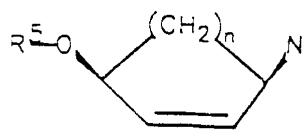

Formel V

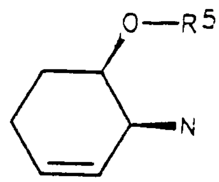

Formel VI

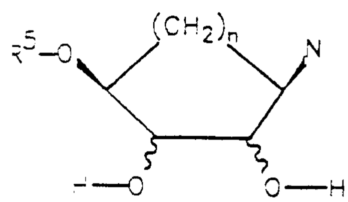

Formel VII

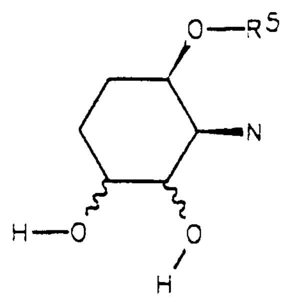

Formel VIII

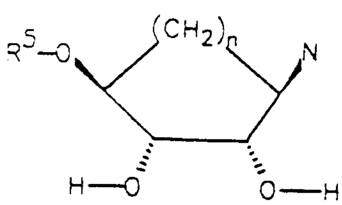

Formel VIIa

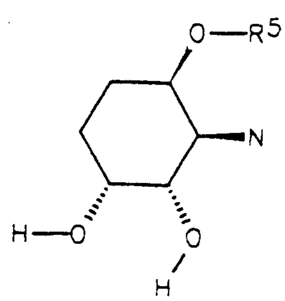

Formel VIIIa

in denen $R^4$ Wasserstoff, C1-C9-Trialkylsilyl, bevorzugt Trimethylsilyl, C1-C6-Alkyl oder C1-C6-Acyl, bevorzugt Acetyl ist und $R^5$ die gleiche Bedeutung wie $R^4$ hat oder C1-C6-, vorzugsweise C1-C3-Alkyl, Benzyl, Trityl oder Benzyloxymethyl ist, und N ein stickstoffhaltiger Heterocyclus wie in den Ansprüchen 1 - 5 definiert ist, und wobei vorhandene Amino-, Hydroxy- und Mercapto-Gruppen am Heterocyclus und weitere NH-Gruppen im Heterocyclus mit Trimethylsilyl, C1-C6-Acyl, Benzyl, Trityl, Benzyloxymethyl, Dimethylaminomethyliden oder N-Methyl-2-pyrrolidinyliden substituiert sein können, mit der Maßgabe, daß in Verbindungen der Formeln III und V $R^4$ und $R^5$ nicht Wasserstoff sind, wenn n = 1 und N = Adenin-9-yl, Thymin-1-yl, Cytosin-1-yl, $N^4$-Dimethylaminomethyliden-cytosin-1-yl, 6-Benzyloxy-2-aminopurin-9-yl, 6-Benzyloxy-2-monomethoxytritylaminopurin-9-yl oder Pyrimidin-2(1H)-on-1-yl ist und daß in Verbindungen der Formel VIIa $R^5$ nicht Wasserstoff ist, wenn n = 1 und N =

Adenin-9-yl ist.

**10.** Verwendung von Verbindungen der Formeln I und II gemäß einem oder mehreren der Ansprüche 1 - 5, wobei die Verbindung der Formel C nicht ausgenommen ist, zur Prophylaxe oder Behandlung von Viruserkrankungen und Erkrankungen, die durch einzellige Parasiten verursacht werden.

**11.** Arzneimittel mit einem Gehalt an mindestens einer der Verbindungen der Formeln I oder II gemäß einem oder mehreren der Ansprüche 1 bis 5, wobei die Verbindung der Formel C nicht ausgenommen ist.

**12.** Verwendung von Verbindungen der Formeln I oder II gemäß einem oder mehreren der Ansprüche 1 - 5, wobei die Verbindung der Formel C nicht ausgenommen ist, zur Herstellung von Arzneimitteln zur Prophylaxe oder Behandlung von Viruserkrankungen oder von Erkrankungen, die durch einzellige Parasiten verursacht werden.

**Patentansprüche für folgende Vertragsstaaten : ES, GR**

**1.** Verfahren zur Herstellung von Verbindungen der Formeln I und II,

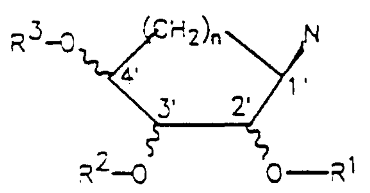

Formel I

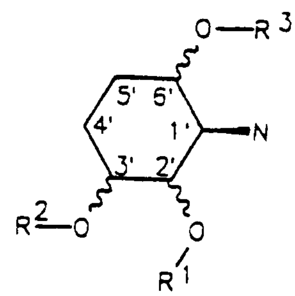

Formel II

in denen N ein über Stickstoff an C-1'gebundener mono- oder bicyclischer Heterocyclus mit mindestens einem Stickstoffatom ist, der unabhängig voneinander mit bis zu drei Amino-, Hydroxy-, Mercapto- und/oder Halogengruppen substituiert sein kann und wobei die Amino-, Hydroxy- und/oder Mercaptogruppen unabhängig voneinander bis zu zweifach mit C1-C6-Alkyl, C1-C6-Acyl-, Benzyl-, Trityl-, Benzyloxymethyl-, Amidingruppen und/oder Phenyl ggfs. seinerseits substituiert mit Halogen, C1-C6-Alkyl, C1-C6-Alkoxy, Amino, C1-C6-Alkylamino oder C1-C12-Dialkylamino substituiert sein können und wobei NH-Funktionen des Heterocyclus mit C1-C6-Acyl-, Benzyl- oder Benzyloxymethylgruppen substituiert sein können, und

n 1 - 4 ist,

und

$R^1$, $R^2$ und $R^3$ unabhängig voneinander Wasserstoff, C1-C8-Alkyl, C3-C8-Alkenyl, C3-C8-Alkinyl, C3-C8-Cycloalkyl, C3-C8-Cycloalkenyl, Benzyl, Trityl, Benzyloxymethyl oder C1-C8-Acyl sind,

oder

$R^1$ und $R^2$ Teil eines 1,3-Dioxa-pentanringes sind, in dessen 2-Position ein oder zwei Wasserstoffatome, unabhängig voneinander mit C1-C8-Alkyl, C2-C16-Dialkyl, C3-C8-Cycloalkyl, C3-C8-Cycloalkenyl, Phenyl, Diphenyl, C1-C8-Alkoxy, C2-C16-Dialkoxy, Dimethylamino, Sauerstoff oder Schwefel substituiert sein können, oder Teil eines cyclischen Sulfits, cyclischen Sulfats, cyclischen Phosphits oder cyclischen Phosphats sind, oder beide Wasserstoffatome ersetzt sind durch die Gruppen - $CH_2$-$CH_2$-$CH_2$-N($CH_3$)-

und

$R^3$ wie oben angegeben ist,

sowie deren physiologisch verträgliche Salze und offensichtliche chemische Äquivalente, wobei die Verbindung der Formel C

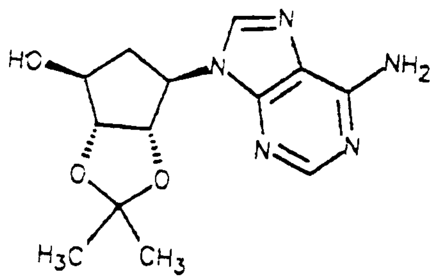

C

ausgenommen ist, dadurch gekennzeichnet, Verbindungen der Formel V oder VI,

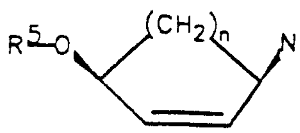

Formel V

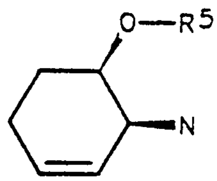

Formel VI

wobei $R^5$ = $R^4$, Alkyl, Benzyl, Trityl oder Benzyloxymethyl ist, und N ein stickstoffhaltiger Heterocyclus wie oben definiert, ist, und wobei eventuell vorhandene Amino-, Hydroxy- und Mercapto-Gruppen am Heterocyclus und weitere NH-Gruppen im Heterocyclus mit Trimethylsilyl, C1-C6-Alkyl, C1-C6-Acyl, Benzyl, Trityl, Benzyloxymethyl, Dimethylaminomethyliden oder N-Methyl-2-pyrrolidinyliden substituiert sein können, mit N-Methyl-morpholin-N-oxid und einer katalytischen Menge Osmiumtetroxid in Tetrahydrofuran, Aceton oder Wasser oder Gemischen umgesetzt wird, wobei Verbindungen der Formeln VII und VIII,

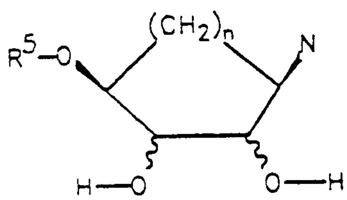

Formel VII

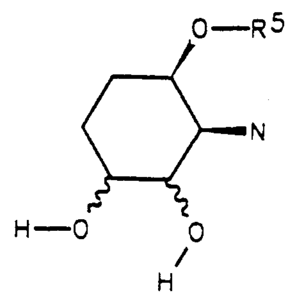

Formel VIII

und daß Verbindungen der Formeln VII und VIII, ihrerseits durch Standardoperationen in Verbindungen der Formeln I und II, wobei die Substituenten N, $R^1$, $R^2$ und $R^3$ die genannten Bedeutungen haben, umgewandelt werden.

**2.** Verfahren gemäß Anspruch 1, dadurch gekennzeichnet, daß Verbindungen der Formeln Ia und IIa

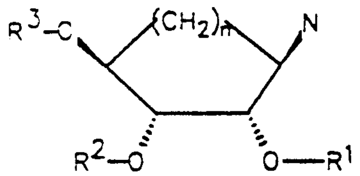

Formel Ia

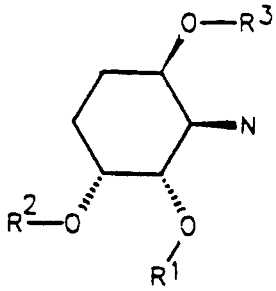

Formel IIa

in denen die Substituenten N, $R^1$, $R^2$ und $R^3$ sowie n wie in Anspruch 1 angegeben sind, hergestellt werden.

**3.** Verfahren gemäß Anspruch 2, dadurch gekennzeichnet, daß Verbindungen der Formeln Ia und IIa, in denen N ein über Stickstoff an C-1' gebundenes Purin, Pyrimidin, 8-Azapurin, 1-Deazapurin, 3-Deazapurin, Benzimidazol, Imidazo[4,5-d]pyridazin, Pyridazin, Pyridin, Triazol, Imidazol oder Imid ist, das unabhängig voneinander mit bis zu drei Amino-, Hydroxy-, Mercapto- und/oder Halogengruppen substituiert sein kann und wobei die Amino-, Hydroxy- und/oder Mercaptogruppen unabhängig voneinander bis zu zweifach mit C1-C6-Alkyl-, C1-C6-Acyl-, Benzyl-, Trityl-, Benzyloxymethyl-, Amidingruppen und/oder Phenyl ggfs. seinerseits substituiert mit Halogen, C1-C6-Alkyl, C1-C6-Alkoxy, Amino, C1-C6-Alkylamino oder C1-C12-Dialkylamino substituiert sein können und wobei NH-Funktionen des Heterocyclus mit C1-C3-Acyl-, Benzyl- oder Benzyloxymethylgruppen substituiert sein können,

und

n 1 - 3 ist,

und

$R^1$, $R^2$ und $R^3$ unabhängig voneinander Wasserstoff, C1-C5-Alkyl, C3-C5-Alkenyl, C3-C5-Alkinyl, C3-C5-Cycloalkyl, C3-C5-Cycloalkenyl, Benzyl, Trityl, Benzyloxymethyl oder C1-C7-Acyl sind,

oder

$R^1$ und $R^2$ Teil eines 1,3-Dioxa-pentanringes sind, in dessen 2-Position ein oder zwei Wasserstoffatome, unabhängig voneinander mit C1-C8-Alkyl, C2-C8-Dialkyl, C3-C8-Cycloalkyl, C3-C8-Cycloalkenyl, Phenyl, Diphenyl, C1-C4-Alkoxy, C2-C8-Dialkoxy,

Dimethylamino, Sauerstoff oder Schwefel substituiert sein können, oder Teil eines cyclischen Sulfits, cyclischen Sulfats, cyclischen Phosphits oder cyclischen Phosphats sind, oder beide Wasserstoffatome ersetzt sind durch die Gruppe $-CH_2-CH_2-CH_2-N(CH_3)-$

und

$R^3$ wie oben angegeben ist, sowie deren physiologisch verträgliche Salze und offensichtliche chemische Äquivalente hergestellt werden.

**4.** Verfahren gemäß den Ansprüchen 1-3, dadurch gekennzeichnet, daß Verbindungen der Formeln Ia und IIa gemäß einem oder mehreren der Ansprüche 1 bis 3, in denen N ein über Stickstoff an C-1' gebundenes Purin, Pyrimidin, 3-Deazapurin, Imidazo[4,5-d]pyridazin, Pyridazin, Pyridin, Imidazol oder Imid ist, das unabhängig voneinander mit bis zu drei Amino-, Hydroxy-, Mercapto- und/oder Halogengruppen substituiert sein kann und wobei die Amino-, Hydroxy- und/oder Mercaptogruppen unabhängig voneinander bis zu zweifach mit C1-C3-Alkyl-, C1-C3-Acyl-, Benzyl-, Trityl-, Amidingruppen und/oder Phenyl ggfs. seinerseits substituiert mit Halogen, C1-C3-Alkyl, C1-C3-Alkoxy, Amino, C1-C3-Alkylamino oder C1-C6-Dialkylamino substituiert sein können,

und

n 1 oder 2 ist

und

$R^1$, $R^2$ und $R^3$ unabhängig voneinander Wasserstoff, C1-C3-Alkyl, Allyl, Propargyl, Cyclopentyl, Benzyl, Trityl, Benzyloxymethyl oder C1-C4-Acyl sind,

oder

$R^1$ und $R^2$ Teil eines 1,3-Dioxa-pentanringes sind, in dessen 2-Position ein oder zwei Wasserstoffatome, unabhängig voneinander mit C1-C3-Alkyl, C2-C6-Dialkyl, C4-C6-Cycloalkyl, C4-C5-Cycloalkenyl, Phenyl, Diphenyl, C1-C3-Alkoxy, C2-C6-Dialkoxy, Sauerstoff oder Schwefel substituiert sein können, oder Teil eines cyclischen Sulfits, cyclischen Sulfats, cyclischen Phosphits oder cyclischen Phosphats sind,

und

$R^3$ wie oben angegeben ist, sowie deren physiologisch verträgliche Salze und offensichtliche chemische Äquivalente hergestellt werden.

5. Verfahren gemäß den Ansprüchen 1-4, dadurch gekennzeichnet, daß Verbindungen der Formel Ia und IIa gemäß einem oder mehreren der Ansprüche 1 bis 4, in denen N ein über Stickstoff an C-1' gebundenes Purin, Pyrimidin, 3-Deazapurin, Pyridazin oder Imidazol ist, das unabhängig voneinander mit bis zu drei Amino-, Hydroxy-, Mercapto- und/oder Halogengruppen substituiert ist und wobei die Amino-, Hydroxy- und/oder Mercaptogruppen unabhängig voneinander bis zu zweifacn mit C1-C3-Alkyl-, C1-C3-Acyl-, Benzylgruppen und/oder Phenyl ggfs. seinerseits substituiert mit Chlor, C1-C3-Alkyl, C1-C3-Alkoxy oder Amino substituiert sein können,

und

n 2 ist

und

$R^1$, $R^2$ und $R^3$ unabhängig voneinander Wasserstoff, C1-C3 -Alkyl, Allyl, Propargyl, Cyclopentyl, Benzyl, Trityl, Benzyloxymethyl oder C1-C4-Acyl sind,

oder

$R^1$ und $R^2$ Teil eines 1,3-Dioxa-pentanringes sind, in dessen 2-Position ein oder zwei Wasserstoffatome, unabhängig voneinander mit C1-C3-Alkyl, Cyclopentyl, Phenyl, Diphenyl, C1-C3-Alkoxy, Sauerstoff oder Schwefel substituiert sein können,

und

$R^3$ wie oben angegeben ist, sowie deren physiologisch verträgliche Salze und offensichtliche chemische Äquivalente hergestellt werden.

6. Verwendung von Verbindungen der Formeln Ia und IIa, gemäß einem oder mehreren der Ansprüche 1 - 5 wobei die Verbindung der Formel C nicht außgenommen ist, sowie deren physiologisch verträgliche Salze und offensichtliche chemische Äquivalente zur Prophylaxe oder Behandlung von Erkrankungen, die durch Viren oder einzellige Parasiten verursacht werden.

7. Verfahren zur Herstellung von Verbindungen der Formeln III und IV,

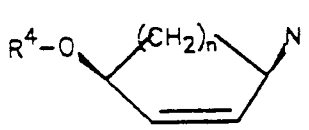

Formel III

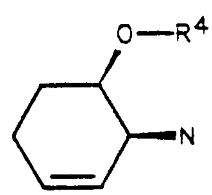

Formel IV

dadurch gekennzeichnet, daß ein Äquivalent eines stickstoffhaltigen Heterocyclus, der wie in den Ansprüchen 1-5 definiert ist, mit mindestens einer freien NH-Funktion oder mindestens einer silylierten, bevorzugt trimethylsilylierten, oder mindestens einer acylierten, bevorzugt acetylierten, N-Funktion im

Heterocyclus, und wobei weitere NH-Funktionen im und Amino-, Hydroxy- und Mercapto-Funktionen am Heterocyclus mit Trimethylsilyl, Acyl, Benzyl, Benzyloxymethyl, Dimethylaminomethyliden oder N-Methyl-2-pyrrolidinyliden substituiert sein können, mit 0.001 bis 0.5 Äquivalenten, bevorzugt 0.005 - 0.1 Äquivalenten eines Palladium-(0)-Katalysators wie Tetrakis(triphenylphosphin)palladium(0) oder Tetrakis(triisopropylphospit)palladium(0), bevorzugt Tetrakis(triisopropylphosphit)palladium(0), welcher in situ aus Palladium-II-acetat, dem jeweiligen Phosphin oder Phosphit und Butyllithium hergestellt werden kann, und mit 1 Äquivalent 1,2-Epoxycycloalk-3-en, bevorzugt Epoxycyclo-penten oder 1,2-Epoxy-cyclohex-3-en, in einem Lösungsmittel wie Tetrahydrofuran, Toluol,Dimethylsulfoxid, Dimethylformamid oder N-Methylpyrrolidon-(2) oder Gemischen davon, bevorzugt in Tetrahydrofuran, Dimethylsulfoxid oder Dimethylformamid oder Gemischen davon bei 0°C bis 110°C, bevorzugt anfangs bei Raumtemperatur und später bei 80 - 90°C unter einer Schutzgasatmosphäre, beispielsweise Argon, während 2 bis 24 Stunden, bevorzugt während 2 - 10 Stunden, zu Verbindungen der Formeln III oder/und IV umgesetzt wird, wobei $R^4$ Wasserstoff ist, wenn in der obigen Reaktion ein stickstoffhaltiger Heterocyclus mit mindestens einer nicht modifizierten NH-Funktion eingesetzt wird oder $R^4$ Trialkylsilyl, bevorzugt Trimethylsilyl, ist, wenn in der obigen Reaktion ein stickstoffhaltiger Heterocyclus, dessen NH-Funktion trialkylsilyl-, bevorzugt trimethylsilylmodifiziert, war.

**8.** Verwendung von Verbindungen der Formeln I und II gemäß einem oder mehreren der Ansprüche 1 - 5, wobei die Verbindung der Formel C nicht ausgenommen ist, zur Prophylaxe oder Behandlung von Viruserkrankungen und Erkrankungen, die durch einzellige Parasiten verursacht werden.

**9.** Arzneimittel mit einem Gehalt an mindestens einer der Verbindungen der Formeln I oder II gemäß einem oder mehreren der Ansprüche 1 bis 5, wobei die Verbindung der Formel C nicht ausgenommen ist.

**10.** Verwendung von Verbindungen der Formeln I und II gemäß einem oder mehreren der Ansprüche 1 - 5, wobei die Verbindung der Formel C nicht ausgenommen ist, zur Herstellung von Arzneimitteln zur Prophylaxe oder Behandlung von Viruserkrankungen oder von Erkrankungen, die durch einzellige Parasiten verursacht werden.